# EUROPEAN PATENT APPLICATION

(11) **EP 4 652 995 A1**
(43) Date of publication of application: **26.11.2025**
(21) Application number: 24744640.4
(22) Date of filing: 16.01.2024
(51) Int. Cl.: A61K 31/4184, A01N 37/40, A01N 43/52, A01P 3/00, A61K 31/609, A61P 31/04

(54) **ANTIBACTERIAL COMPOSITION, PHARMACEUTICAL AGENT, AND ANTIBACTERIAL METHOD**

(30) Priority: 16.01.2023 JP 2023004711
(71) Applicant: Japan Science and Technology Agency, Kawaguchi-shi, Saitama 332-0012 (JP)
(72) Inventor: MURATA Takeshi, Chiba-shi, Chiba 263-8522 (JP); ABE Shohei, Chiba-shi, Chiba 263-8522 (JP); SUZUKI Kano, Chiba-shi, Chiba 263-8522 (JP); GOTO Yoshiyuki, Chiba-shi, Chiba 263-8522 (JP)
(74) Representative: Grünecker Patent- und Rechtsanwälte PartG mbB
(86) International application number: PCT/JP2024/000914
(87) International publication number: WO 2024/154719

(57) **Abstract**

The present invention provides an antibacterial composition containing a 2-phenylbenzimidazole-based compound and a salicylanilide-based compound. In addition, the present invention provides an antibacterial method for inhibiting proliferation of bacteria using the antibacterial composition, the method including: administering the 2-phenylbenzimidazole-based compound and the salicylanilide-based compound to bacteria having Na⁺-transporting V-ATPase and F-ATPase; and binding the 2-phenylbenzimidazole-based compound to the V-ATPase of the bacteria to inhibit activity of the V-ATPase, and breaking a proton concentration gradient by the F-ATPase with the salicylanilide-based compound to inhibit activity of the F-ATPase.

## Description

### Technical Field

The present invention relates to an antibacterial composition, a pharmaceutical reagent, and an antibacterial method.

### Background Art

V-ATPase is a supermolecular complex that exists in an organelle membrane in eukaryotes, and has a subunit structure complex. The V-ATPase has a function as an ion-transporting molecular motor, and transports ions between the inside and outside of the membrane while rotating a specific detail unit in the membrane by ATP hydrolysis energy. The V-ATPase is known to exist also in prokaryotes (bacteria), and may also be referred to as A-ATPase.

The V-ATPase is required to allow cells to grow in a specific environment. For example, in "Enterococcus hirae" which is enterococcus, the V-ATPase has a function to hydrolyze ATP and to transfer intracellular sodium ions (Na⁺) to the outside of the cell, and by such a function, enterococcus can grow under high salt concentration or high-pH (alkaline) conditions.

In addition, the V-ATPase that is similar to that in enterococcus exists in a variety of pathogenic microorganisms, and plays an important role in the proliferation under an alkaline condition. Accordingly, a compound that inhibits an ion transport function of V-ATPase is useful as an antibacterial agent for pathogenic bacteria that cause diseases, and is expected as a new therapeutic agent.

The present inventors have hitherto found that a 2-phenylbenzimidazole-based compound exhibits an activity inhibitory function of Na⁺-transporting V-ATPase (see, for example, Patent Literature 1). According to this literature, the use of the 2-phenylbenzimidazole-based compound as a V-ATPase activity inhibitor can selectively and efficiently inhibit proliferation of bacteria that cause diseases and the like. In addition, examples in this literature illustrate that the 2-phenylbenzimidazole-based compound is effective in inhibiting the proliferation of Enterococcus faecalis (E. faecalis), Enterococcus faecium (E. faecium), and Clostridium difficile (C. difficile) of vancomycin-resistant enterococci (VRE).

### Citation List

### Patent Literature

Patent Literature 1: WO 2020/149295 A (claims 1 and 2, and the like)

### Summary of Invention

### Technical Problem

As described in this literature, expression of V-ATPase in bacteria depends on an external environment such as a pH, and for example, in E. hirae, V-ATPase is not expressed near a neutral pH but is expressed at an alkaline or high salt concentration. The 2-phenylbenzimidazole-based compound is effective as an antibacterial agent in an environment where V-ATPase is expressed, such as alkaline or in the small intestine, but is less effective as an antibacterial agent in an environment where V-ATPase is not expressed, such as acidic or neutral or in other organs. Therefore, an antibacterial agent effective in various environments is required.

An object of the present invention is to provide an antibacterial composition effective in various environments including acidic and neutral environments, a pharmaceutical reagent containing the antibacterial composition, and an antibacterial method using the antibacterial composition.

### Solution to Problem

The present inventors have intensively studied to solve the problems. As a result, the present inventors have found that when a 2-phenylbenzimidazole-based compound and a salicylanilide-based compound are used in combination, the compounds have antibacterial activity in various environments including acidic and neutral environments, thereby completing the present invention.

[1] An antibacterial composition comprising a 2-phenylbenzimidazole-based compound and a salicylanilide-based compound.
   An antibacterial composition comprising a Na+-transporting V-ATPase activity inhibitor containing a 2-phenylbenzimidazole-based compound, and a protonophore containing a salicylanilide-based compound.
[2] The antibacterial composition according to [1] or [1'], wherein the 2-phenylbenzimidazole-based compound is a compound represented by the following Formula (1). 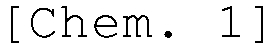 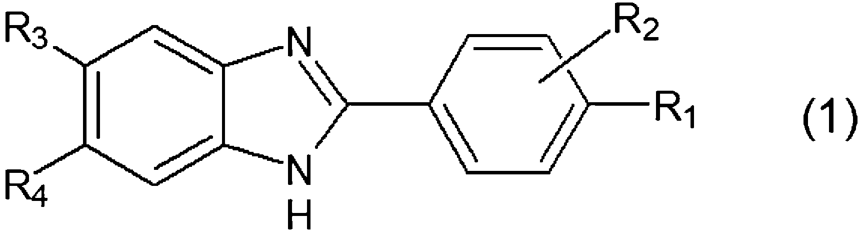 wherein R₁ is selected from a hydroxy group, an alkyl group having 1 to 10 carbon atoms, an alkoxy group having 1 to 10 carbon atoms, and a haloalkoxy group having 1 to 3 carbon atoms, each bonded to an adjacent phenyl group via oxygen, or selected from a dialkylamino group having 1 to 10 carbon atoms of each alkyl group, a heterocyclic amine having 2 to 6 carbon atoms, and a carboxylic acid amide group which may have a substituent bonded to a carbon atom, each bonded to an adjacent phenyl group via nitrogen, or represents bromine, iodine, or a straight-chain hydrocarbon group having 2 to 5 carbon atoms. R₂ represents hydrogen or a haloalkoxy group having 1 to 3 carbon atoms. R₃ and R₄ represent a hydrogen atom, an alkyl group having 1 to 4 carbon atoms, or a halogen atom selected from fluorine, chlorine, bromine, and iodine, and R₃ and R₄ may be the same as or different from each other.
[3] The antibacterial composition according to [2], wherein the compound represented by Formula (1) is selected from the group consisting of compounds represented by the following Formulas (1-1) to (1-5). 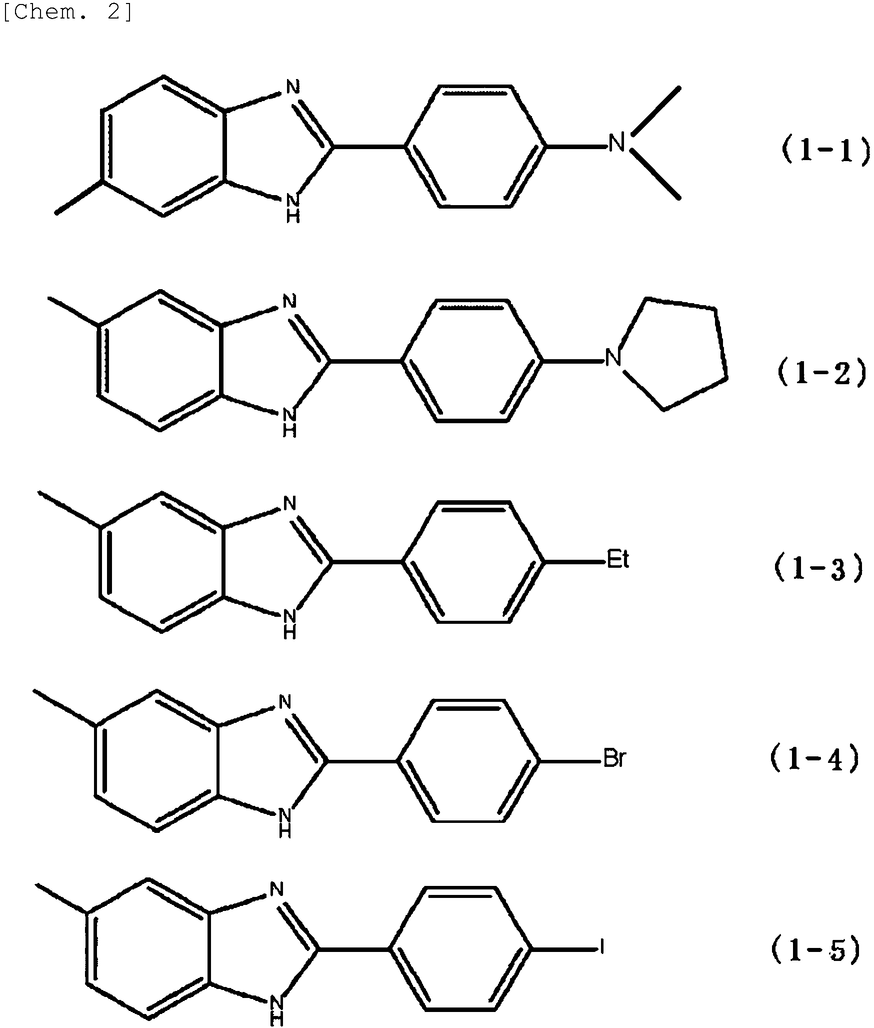
[4] The antibacterial composition according to [1] or [1'], wherein the salicylanilide-based compound is a compound represented by the following Formula (2). 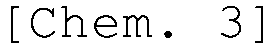 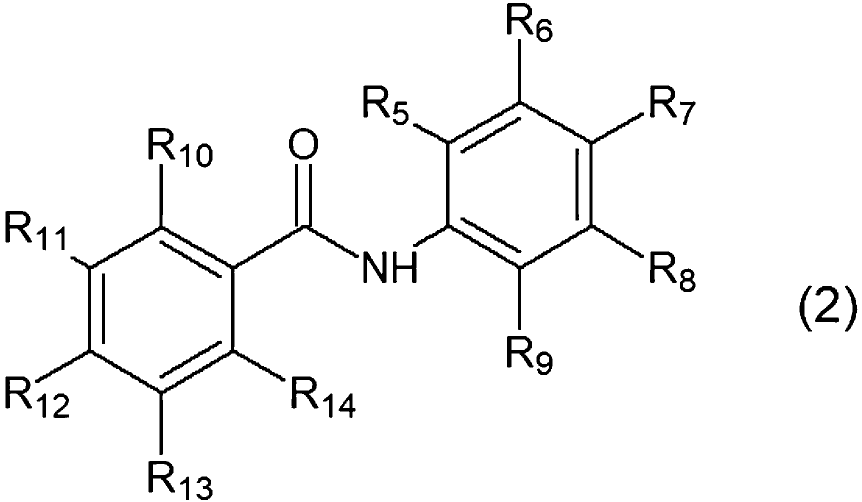 In the formula, R₅ to R₉ represent a hydrogen atom, a nitro group, or a halogen atom selected from fluorine, chlorine, bromine, and iodine, at least one of R₅ to R₉ is a halogen atom, and R₃ to R₉ may be the same as or different from each other. R₁₀ to R₁₄ represent a hydrogen atom, a hydroxyl group, or a halogen atom selected from fluorine, chlorine, bromine, and iodine, at least one of R₁₀ to R₁₄ is a hydroxyl group, at least one is a halogen atom, and R₁₀ to R₁₄ may be the same as or different from each other.
[5] The antibacterial composition according to [4], wherein the compound represented by Formula (2) is niclosamide represented by the following Formula (2-1) or oxyclozanide represented by the following Formula (2-2). 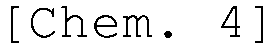 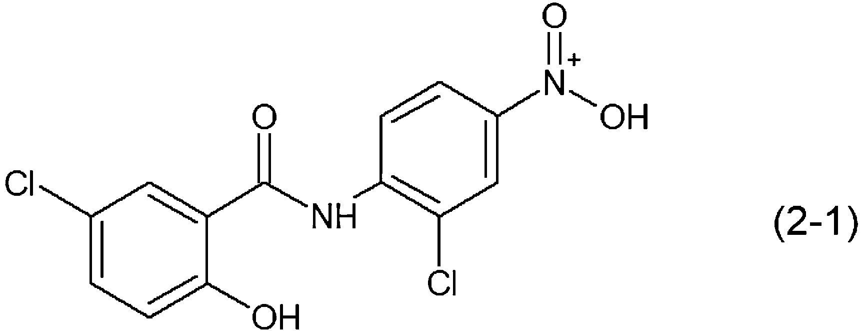 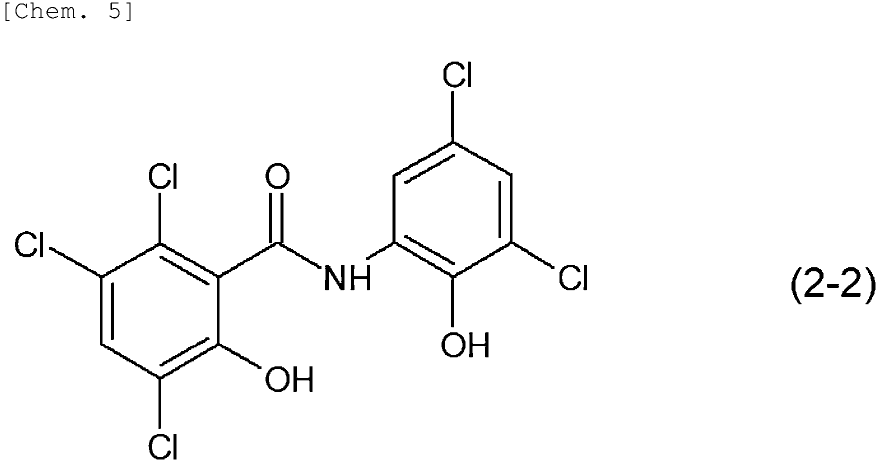
[6] A pharmaceutical reagent containing the antibacterial composition according to [1] or [1'].
[7] An antibacterial method for inhibiting proliferation of bacteria using the antibacterial composition according to [1] or [1'], comprising the steps of:
   administering the 2-phenylbenzimidazole-based compound and the salicylanilide-based compound to bacteria having Na⁺-transporting V-ATPase and F-ATPase; and
   inhibiting activity of the V-ATPase by binding the 2-phenylbenzimidazole-based compound to the V-ATPase of the bacteria, and
   inhibiting activity of the F-ATPase by breaking a proton concentration gradient by the F-ATPase with the salicylanilide-based compound.
[8] The antibacterial method according to [7], wherein the bacteria are drug-resistant enterococci.
[9] Use of a 2-phenylbenzimidazole-based compound in combination with a salicylanilide-based compound.
[10] The use according to [9], wherein the 2-phenylbenzimidazole-based compound is a compound represented by the following Formula (1). 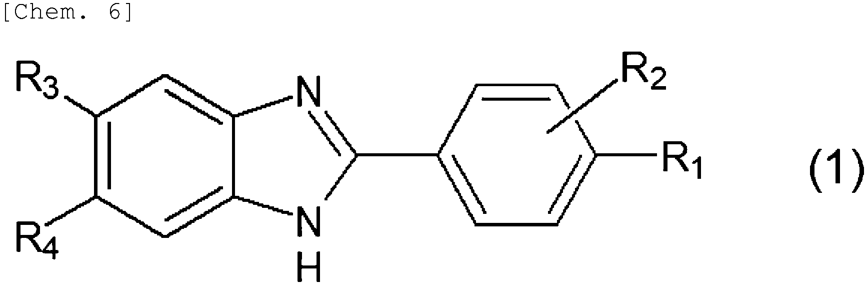
[11] The use according to [10], wherein the compound represented by Formula (1) is selected from the group consisting of compounds represented by the following Formulas (1-1) to (1-5). 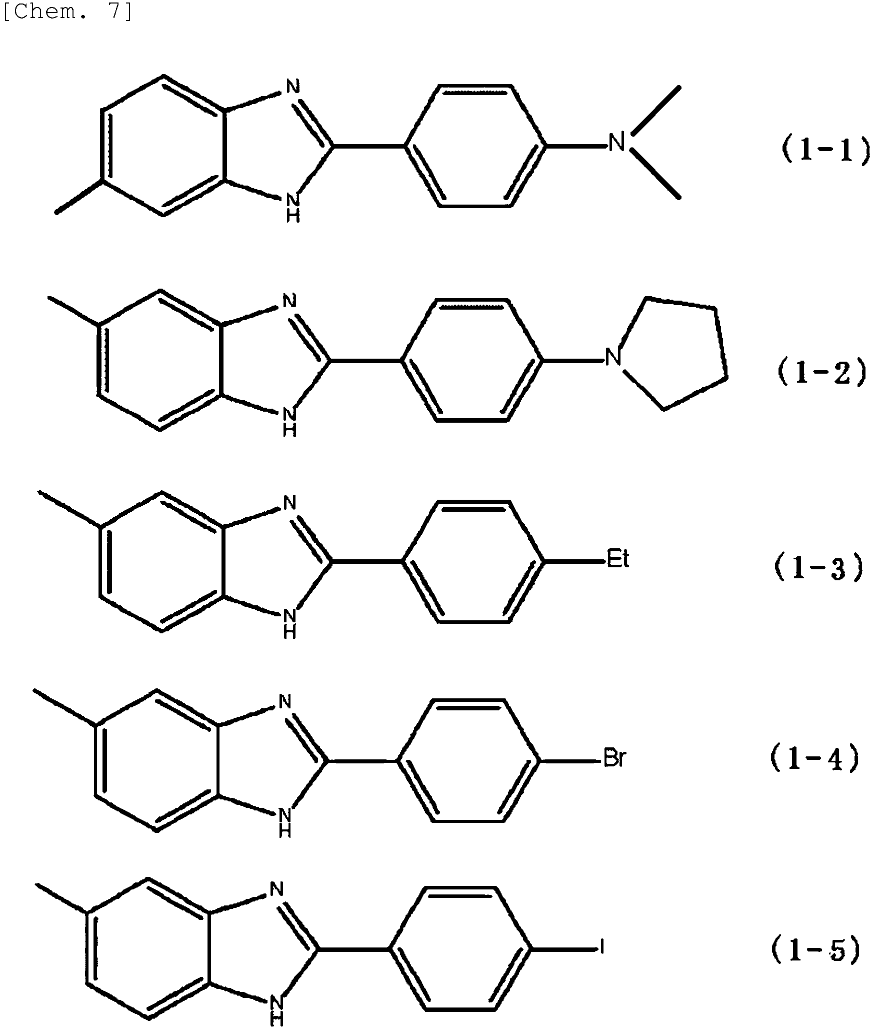
[12] The use according to any one of [9] to [11], wherein the salicylanilide-based compound is a compound represented by the following Formula (2), (2-1), or (2-2). 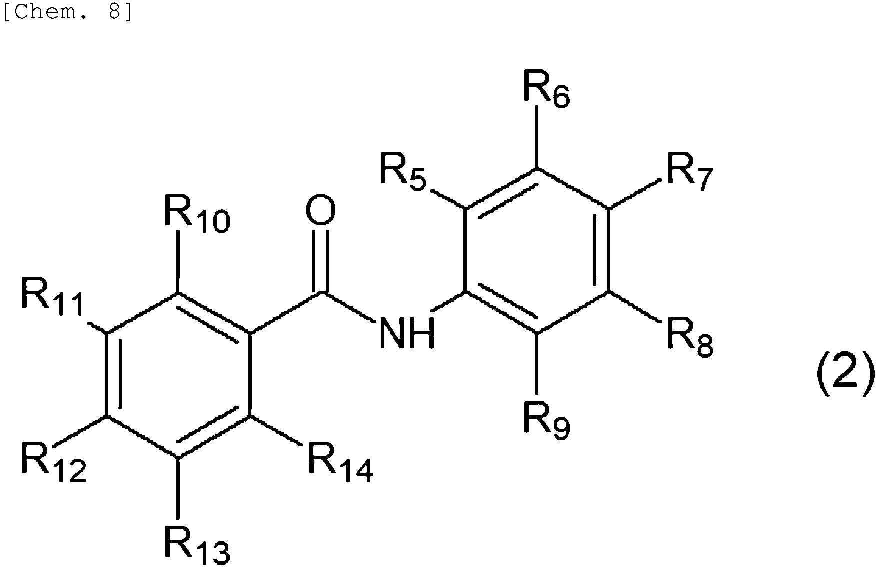 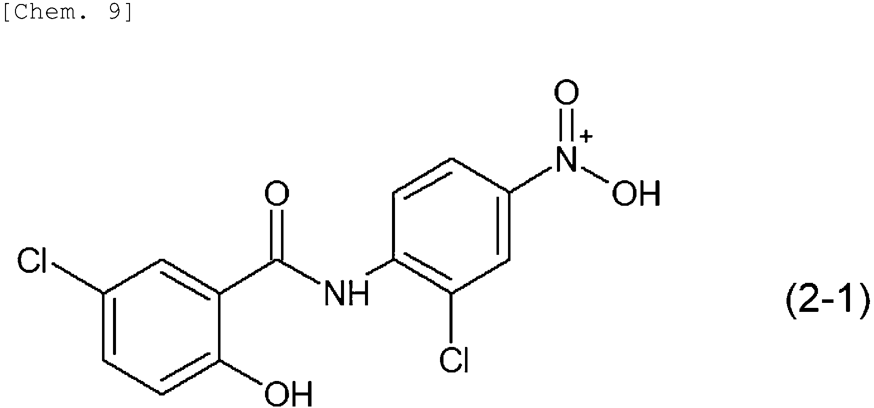 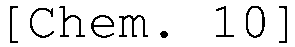 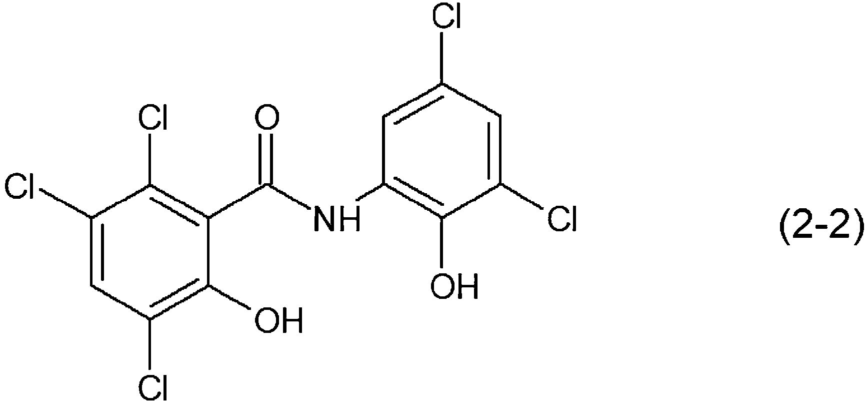
[13] Use of a salicylanilide-based compound in combination with a 2-phenylbenzimidazole-based compound.
[14] The use according to [13], wherein the salicylanilide-based compound is a compound represented by the following Formula (2). 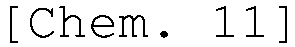 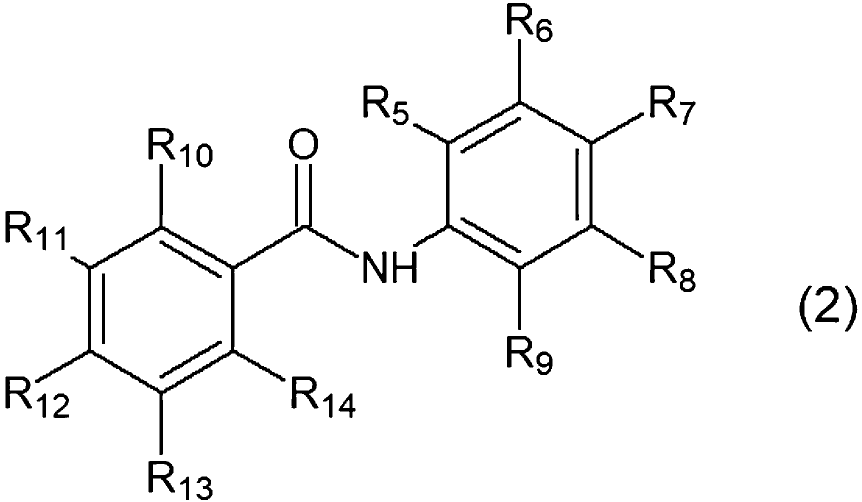
[15] The use according to [14], wherein the compound represented by Formula (2) is niclosamide represented by the following Formula (2-1) or oxyclozanide represented by the following Formula (2-2). 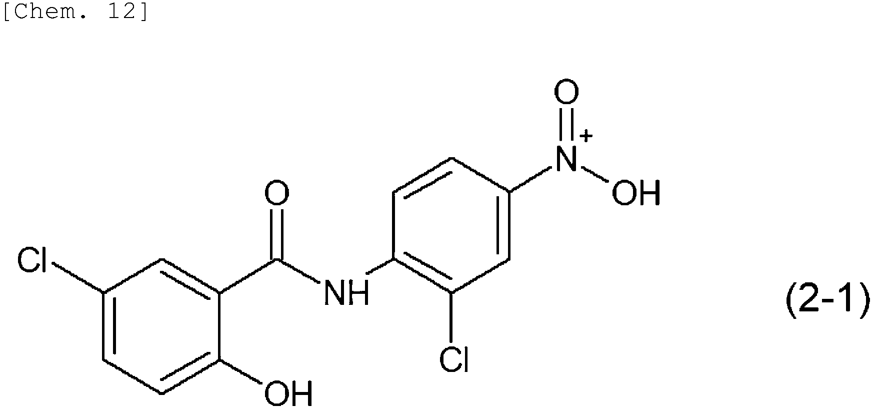 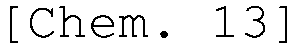 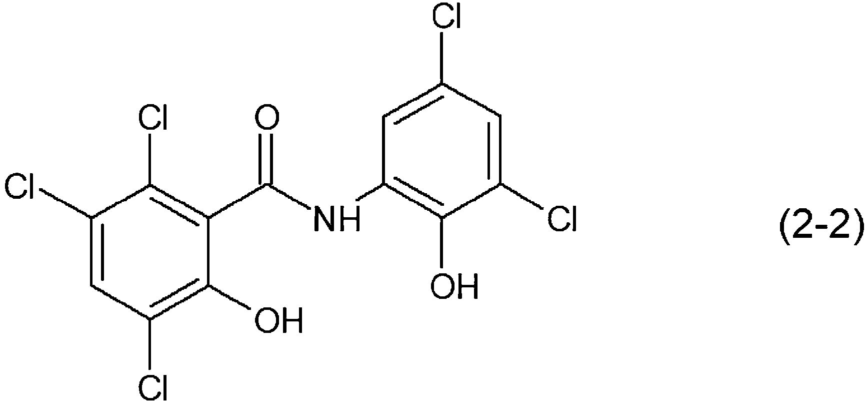
[16] The use according to any one of [13] to [15], wherein the 2-phenylbenzimidazole-based compound is selected from the group consisting of compounds represented by the following Formula (1) or Formulas (1-1) to (1-5).

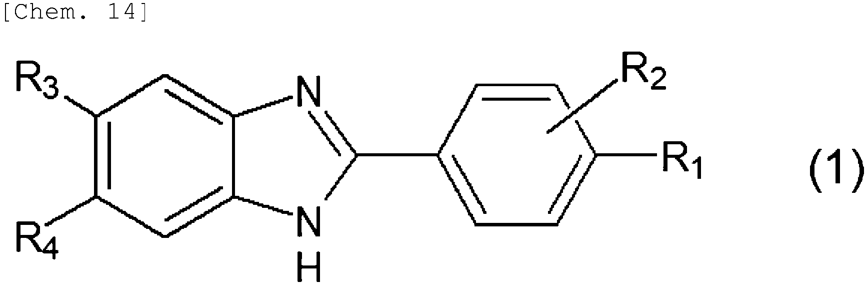

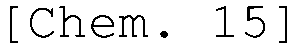

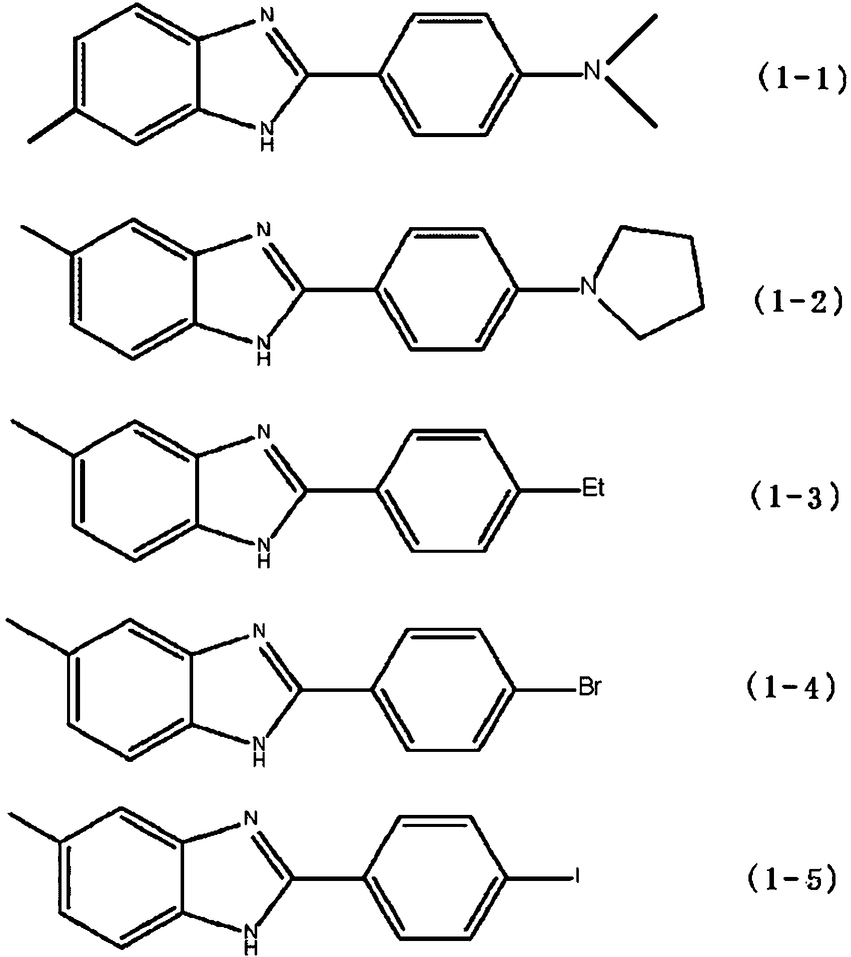

### Advantageous Effects of Invention

According to the present invention, it is possible to provide an antibacterial composition effective in various environments including acidic and neutral environments, a pharmaceutical reagent comprising the antibacterial composition, and an antibacterial method using the antibacterial composition.

### Brief Description of Drawings

Fig. 1 is a schematic diagram showing functions of V-ATPase and F-ATPase in bacteria.
Fig. 2 is a graph showing the results of the in vitro proliferation inhibitory effect of E. faecium using V-161 in combination with niclosamide.
Fig. 3 is a graph showing the results of the in vitro proliferation inhibitory effect of E. faecium in the coexistence of antibiotics.
Fig. 4 is a graph showing the in vitro proliferation inhibitory effect of E. faecium using V-161 or an analogue thereof in combination with niclosamide.
Fig. 5 is a graph showing the results of the in vitro proliferation inhibitory effect of E. faecium using a salicylanilide-based compound in combination with V-161.
Fig. 6 is a graph showing the results of the in vitro proliferation inhibitory effect of C. perfringens and C. difficile in combination use of V-161 and niclosamide.
Fig. 7 is a graph showing the in vivo proliferation inhibitory effect of combination use of V-161 and niclosamide on a mouse to which VRE is pre-administered.

### Description of Embodiments

### 1. Antibacterial composition

The present invention provides an antibacterial composition containing a 2-phenylbenzimidazole-based compound and a salicylanilide-based compound. Hereinafter, each inhibitor will be described in detail.

### (1) 2-Phenylbenzimidazole-based compound (V-ATPase activity inhibitor)

The 2-phenylbenzimidazole-based compound of the present invention has a function of inhibiting the activity of Na⁺-transporting V-ATPase, and has a role as a V-ATPase activity inhibitor. The 2-phenylbenzimidazole-based compound exhibits an antibacterial function mainly in an alkaline region of pH 7.5 or more. Examples of the 2-phenylbenzimidazole-based compound include a compound represented by the following Formula (1). Note that, in the following description, "Na⁺-transporting V-ATPase" may be simply referred to as "V-ATPase".

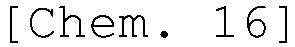

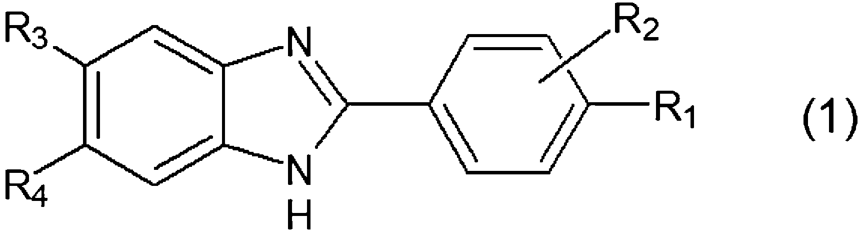

In the formula, R₁ is selected from a hydroxy group, an alkyl group having 1 to 10 carbon atoms, an alkoxy group having 1 to 10 carbon atoms, and a haloalkoxy group having 1 to 3 carbon atoms, each bonded to an adjacent phenyl group via oxygen, or selected from a dialkylamino group having 1 to 10 carbon atoms of each alkyl group, a heterocyclic amine having 2 to 6 carbon atoms, and a carboxylic acid amide group which may have a substituent bonded to a carbon atom, each bonded to an adjacent phenyl group via nitrogen, or represents bromine, iodine, or a straight-chain hydrocarbon group having 2 to 5 carbon atoms. R₂ represents hydrogen or a haloalkoxy group having 1 to 3 carbon atoms. R₃ and R₄ represent a hydrogen atom, an alkyl group having 1 to 4 carbon atoms, or a halogen atom selected from fluorine, chlorine, bromine, and iodine, and R₃ and R₄ may be the same as or different from each other.

In R₁, the hydroxy group is a substituent represented by "-OH". The alkoxy group having 1 to 10 carbon atoms is a substituent represented by "-O-R₁ₐ" (R₁ₐ represents an alkyl group having 1 to 10 carbon atoms). In addition, the haloalkoxy group having 1 to 3 carbon atoms in R₁ is a substituent represented by "-O-R_{1b}-Xₙ₁" (R₁ₐ represents an alkyl group having 1 to 3 carbon atoms, X represents a halogen atom selected from fluorine, chlorine, bromine, and iodine, and n1 = 1 to 3). The dialkylamino group having 1 to 10 carbon atoms of each alkyl group is a substituent represented by "-N(R_{1c})(R_{1d})" (R_{1c} represents an alkyl group having 1 to 10 carbon atoms, R₁₆ represents an alkyl group having 1 to 10 carbon atoms, and R_{1c} and R₁₆ may be the same as or different from each other). The heterocyclic amine having 2 to 6 carbon atoms is a substituent represented by "-N-(CH₂)ₙ₂-" (n2 = 2 to 6). The carboxylic acid amide group is a substituent represented by "-NH-C(=O)-". The bromine is a substituent represented by "-Br", and the iodine is a substituent represented by "-I". The straight-chain hydrocarbon group having 2 to 5 carbon atoms is a substituent represented by "-(CH₂)ₙ₃-CH₃" (n3 = 1 to 4).

In R₂, R₃, and R₄, the hydrogen is a substituent represented by "-H". In R₂, the haloalkoxy group having 1 to 3 carbon atoms is a substituent represented by "-OR₂ₐ-Xₙ₄" (R₂ₐ represents a hydrocarbon group having 1 to 3 carbon atoms, X represents a halogen atom selected from fluorine, chlorine, bromine, and iodine, and n4 = 1 to 3). R₂ may be located at the ortho position or the meta position as viewed from the benzimidazole group in the phenyl group.

In R₃ and R₄, the alkyl group having 1 to 4 carbon atoms is a substituent represented by "-O-R₁ₐ" (R₁ₐ represents an alkyl group having 1 to 4 carbon atoms). In the above, the "halogen atom" is selected from fluorine, chlorine, bromine, and iodine.

Examples of the 2-phenylbenzimidazole-based compound of Formula (1) include compounds represented by the following Formulas (1-1) to (1-5).

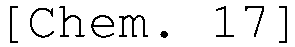

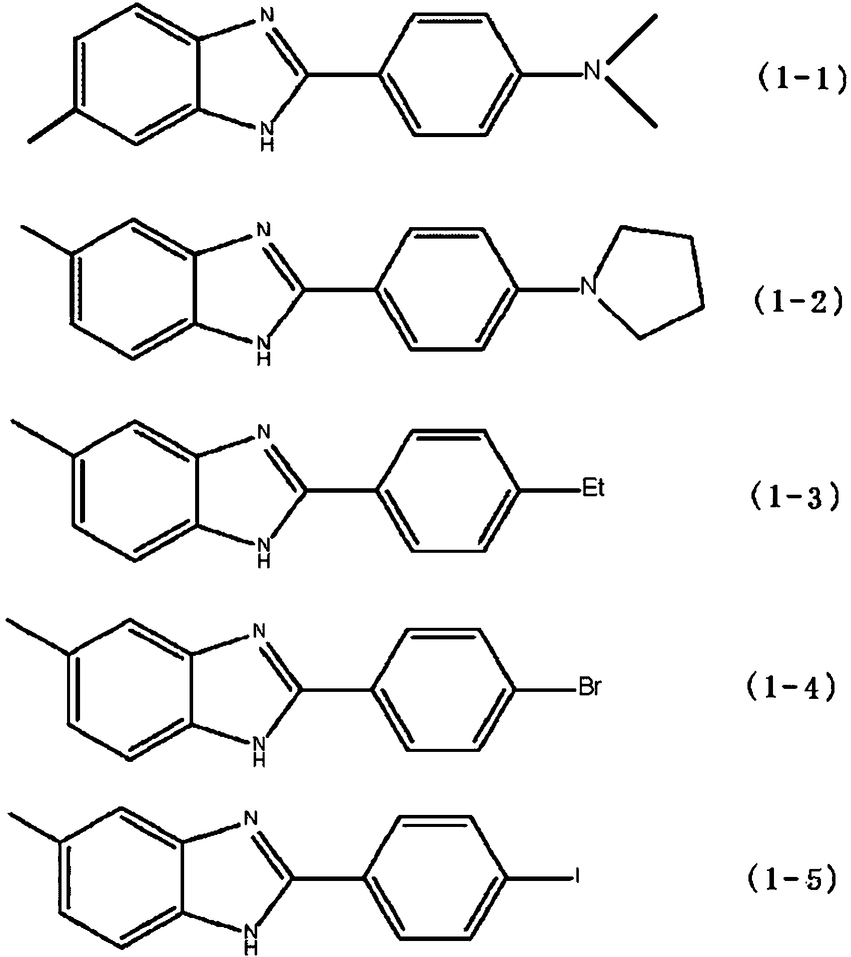

Among them, N,N-dimethyl-4-(6-methyl-1H-benzo[d]imidazol-2-yl)aniline represented by the following Formula (1-1) (Formula V-161 in Examples) is preferable since the effect of V-ATPase activity inhibition is high.

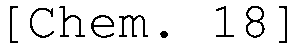

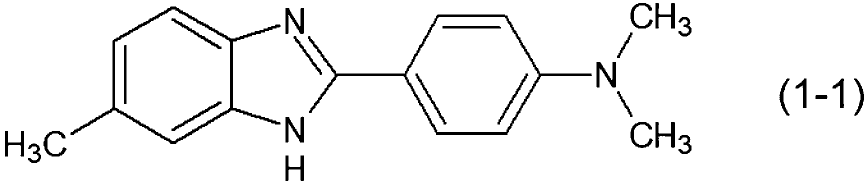

### (2) Salicylanilide-based compound (protonophore)

The salicylanilide-based compound of the present invention has a function of inhibiting the activity of F-ATPase, and has a role as a protonophore. Here, the protonophore means an aromatic compound that transfers a proton through a lipid bilayer. The salicylanilide-based compound exhibits an antibacterial function mainly in an acidic to neutral range of a pH of 7.0 or less. Examples of the salicylanilide-based compound include a compound represented by the following Formula (2) (halogenated salicylanilide-based compound).

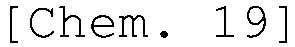

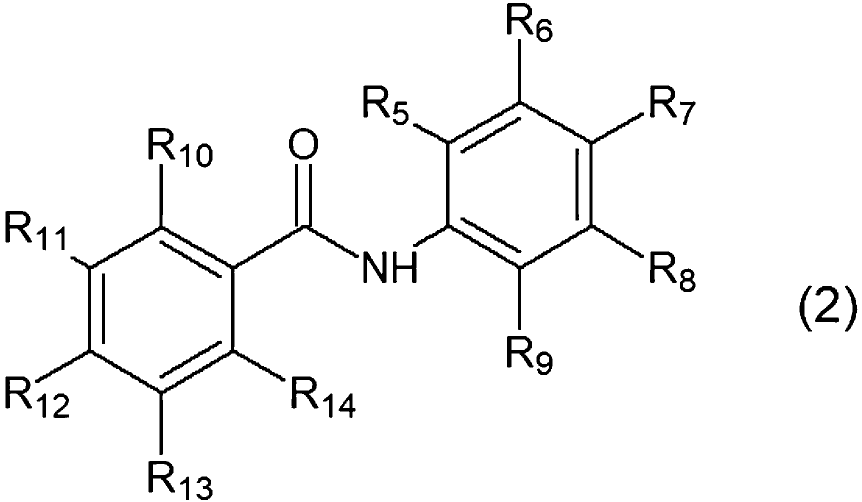

In the formula, R₅ to R₉ represent a hydrogen atom, a nitro group, or a halogen atom selected from fluorine, chlorine, bromine, and iodine, at least one of R₅ to R₉ is a halogen atom, and R₃ to R₉ may be the same as or different from each other. R₁₀ to R₁₄ represent a hydrogen atom, a hydroxyl group, or a halogen atom selected from fluorine, chlorine, bromine, and iodine, at least one of R₁₀ to R₁₄ is a hydroxyl group, at least one is a halogen atom, and R₁₀ to R₁₄ may be the same as or different from each other.

Examples of the salicylanilide-based compound represented by Formula (2) include niclosamide (5-chloro-2-hydroxy-N-(2-chloro-4-nitrophenyl)benzamide), oxyclozanide (2,2'-dihydroxy-3,3',5,5',6-pentachlorobenzanilide), and bromosalicylchloranilide (5-bromo-N-(4-chlorophenyl)-2-hydroxybenzamide).

Among them, niclosamide represented by the following Formula (2-1) or oxyclozanide represented by Formula (2-2) is preferable because the effect of inhibiting the F-ATPase activity is high.

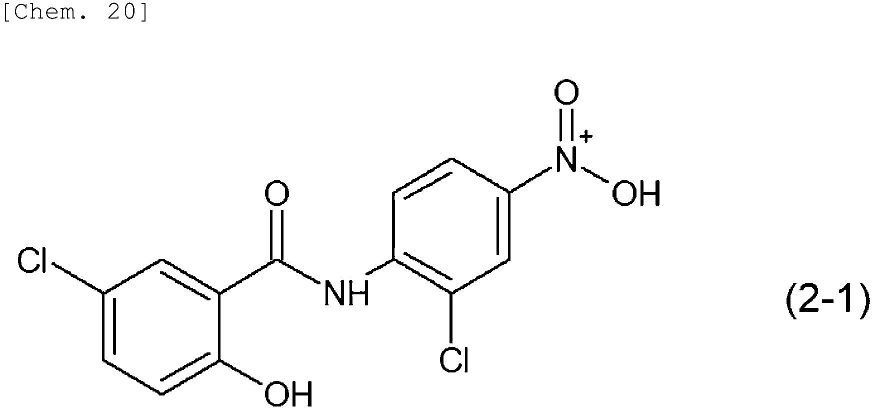

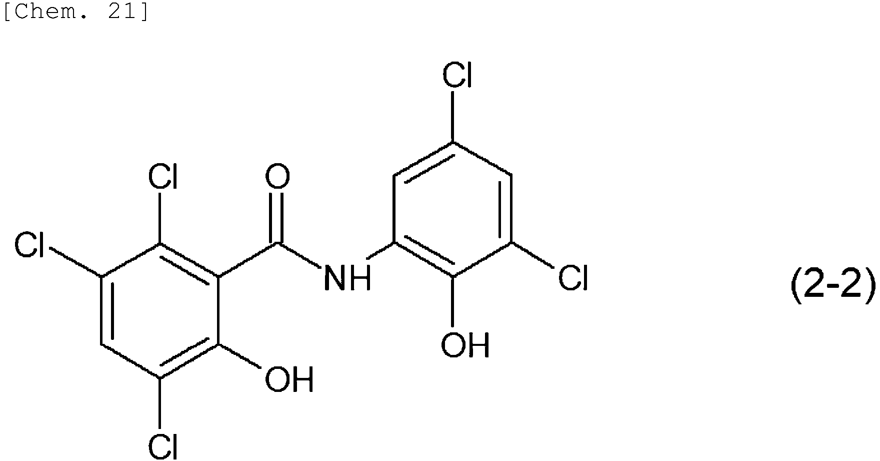

### 2. Mechanism of antibacterial activity

In the present invention, the 2-phenylbenzimidazole-based compound and the salicylanilide-based compound are used in combination to have antibacterial activity over a wide range of pH or salt concentrations. Although the inventors are not bound by any particular theory, an example of the mechanism will be described below.

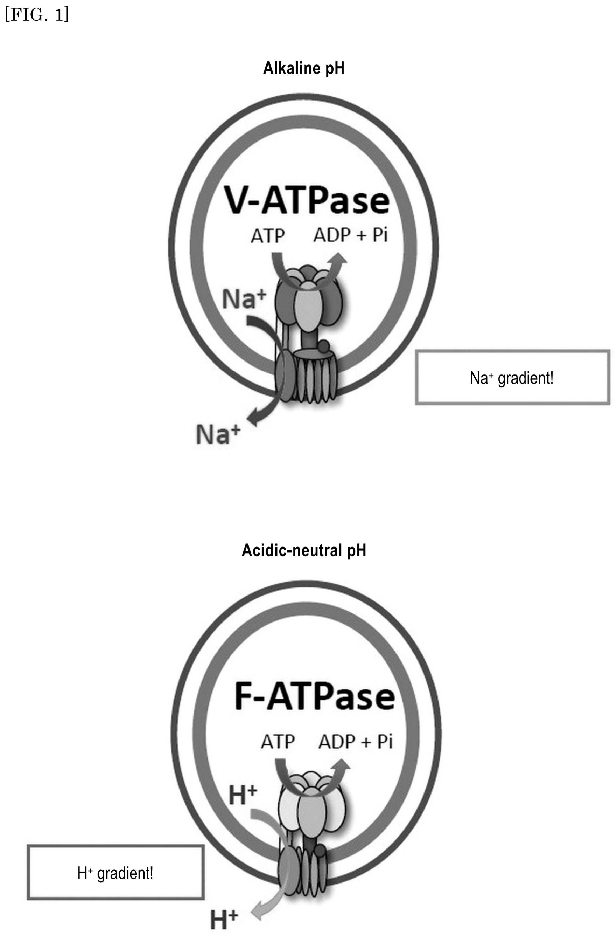

As illustrated in Fig. 1, Na⁺-transporting V-ATPase exists in an organelle membrane of eukaryotes, and degrades ATP to transport sodium ions out of cells. On the other hand, in enterococci such as E. hirae, V-ATPase is not expressed near an acidic to neutral pH (pH ≤ 7.0) or at a low salt concentration, and V-ATPase is expressed at an alkaline pH (pH > 7.0) or at a high salt concentration, and sodium ions in the membrane are discharged to the outside of the membrane. That is, a Na⁺ concentration in a cell such as E. hirae is kept low at an alkaline pH (pH > 7.0) or at a high salt concentration, and proliferation is possible even in an alkaline or high salt concentration environment.

The V-ATPase activity inhibitor has a function of binding to V-ATPase and inhibiting the activity. The 2-phenylbenzimidazole-based compound binds to a membraneembedded rotor ring (c-ring) of V-ATPase and inhibits rotation thereof, thereby inhibiting ion transport. For example, a compound V-161 (Formula (1-1)) of Examples described below binds to two bases (T140 and N615) of both the a-subunit and the c-ring that are important for the activity of Na⁺-transporting V-ATPase. Therefore, V-ATPase cannot discharge sodium ions out of cells and cannot maintain Na⁺ homeostasis, and bacterial proliferation is inhibited. Note that V-161 hardly binds to H⁺-transporting V-ATPase, and specifically acts on Na⁺-transporting V-ATPase.

F-ATPase exists in a plasma membrane of a cell, an inner membrane of mitochondria, and the like, and synthesizes ATP by using a proton concentration gradient inside and outside the membrane as a driving force. On the other hand, F-ATPase existing in enterococci decomposes ATP and discharges a proton outside the cell in acidic to neutral environments, thereby forming a proton concentration gradient inside and outside the cell. Therefore, enterococci are able to grow in an acidic environment. The salicylanilide-based compound binds to a proton and transfers through a lipid bilayer, thereby breaking a proton concentration gradient. As a result, the proton concentration gradient formed by F-ATPase cannot be maintained, and the proliferation of bacteria is inhibited.

The antibacterial composition of the present invention can effectively function to drug-resistant bacteria particularly in the intestine. Bacteria such as lactic acid bacteria that do not have Na⁺-transporting V-ATPase are killed by a drug such as an antibiotic, but these bacteria have a role of maintaining the intestine acidic. Therefore, when the drug is administered to a human, the intestinal environment becomes alkaline due to the death of lactic acid bacteria and the like. Examples of the drug include vancomycin, ampicillin, cefoperazone, and linezolid. Bacteria having Na⁺-transporting V-ATPase can grow even in an alkaline or high salt concentration environment by keeping intracellular Na⁺ low by the function of V-ATPase. Therefore, by administering a Na⁺-transporting V-ATPase activity inhibitor, the proliferation of bacteria having Na⁺-transporting V-ATPase can be inhibited even in an alkaline or high salt concentration environment.

On the other hand, in bacterial blood infection or urinary tract infection, since blood or urine has a neutral or acidic pH (blood has a pH of about 7.0 to 7.5 and urine has a pH of about 6.0), it is difficult to obtain an antibacterial effect only with the 2-phenylbenzimidazole-based compound that functions in an alkaline environment. Therefore, by using the salicylanilide-based compound in combination, the antibacterial activity can be obtained in a wide pH range such as a pH of 5 to 9. In particular, when each of the 2-phenylbenzimidazole-based compound and the salicylanilide-based compound is used alone near a pH of 7.0 to 7.5, it is difficult to obtain the antibacterial effect, but when the 2-phenylbenzimidazole-based compound and the salicylanilide-based compound are used in combination, the bacterial proliferation inhibitory effect is enhanced by a synergistic effect.

The antibacterial composition of the present invention can exhibit an antibacterial function by being directly administered to bacteria. A concentration of the 2-phenylbenzimidazole-based compound to be administered to bacteria can be appropriately determined, and is, for example, in a range of 0.1 µM to 1,000 µM, and preferably in a range of 1 µM to 100 µM. Similarly, a concentration of the salicylanilide-based compound can be appropriately determined, and is, for example, in a range of 0.1 µM to 1,000 µM, and preferably in a range of 1 µM to 100 µM. It is preferable that the antibacterial composition is administered to bacteria and the antibacterial treatment is performed at a temperature in a range of 25 to 50°C, preferably of 30 to 40°C for 1 to 48 hours, and preferably for 10 to 24 hours. In addition, as a ratio of the 2-phenylbenzimidazole-based compound and the salicylanilide-based compound in the antibacterial composition, a weight ratio is in a range of 100:1 to 1:100, preferably in a range of 50:1 to 1:50, and more preferably in a range of 10:1 to 1:10.

### 3. pharmaceutical reagent and antibacterial method

The antibacterial composition of the present invention is useful as an antibacterial agent ( pharmaceutical reagent) for microorganisms expressing V-ATPase and F-ATPase. Examples of the microorganism include, but are not limited to, E. faecium (neonatal meningitis, endocarditis, and the like), C. perfringens (enterotoxemia, gas gangrene, food poisoning, necrotizing enterocolitis, and the like), and C. difficile (pseudomembranous colitis, inflammatory diarrhea, and the like), which are enterobacteria. Even when two or more of these microorganisms coexist, the antibacterial composition of the present invention exhibits a proliferation inhibitory effect against all bacteria. In one non-limiting embodiment of the present invention, the antibacterial composition has an effect on enterococci resistant to a drug such as an antibiotic (drug-resistant enterococci). In another non-limiting emebodiment of the present invention, the antibacterial composition is effective against enterococci having a microorganism expressing V-ATPase, and the like (drug-resistant enterococci).

The antibacterial composition of the present invention is useful as an active ingredient in a drug such as a pharmaceutical reagent or an agrochemicals, and is particularly preferably used for a pharmaceutical reagent. Examples of the dosage form of the pharmaceutical reagent include tablets, capsules, pills, powder, granules, fine granules, a jellies, and liquids.

A concentration of the 2-phenylbenzimidazole-based compound contained in the pharmaceutical reagent can be appropriately determined, and is, for example, in a range of 0.1 µM to 1,000 µM, and preferably in a range of 1 µM to 100 µM. In addition, a concentration of the salicylanilide-based compound contained in the pharmaceutical reagent can be appropriately determined, and is, for example, in a range of 0.1 µM to 1,000 µM, and preferably in a range of 1 µM to 100 µM. Both the 2-phenylbenzimidazole-based compound and the salicylanilide-based compound can be dissolved in a solvent such as water and then used, or can be used in a form of powder.

The pharmaceutical reagent may contain an additive such as a solvent, an excipient, a binding agent, a disintegrant, a lubricating agent, a stabilizer, or a suspending agent within the range of not impairing the effects of the present invention, in addition to the 2-phenylbenzimidazole-based compound and the salicylanilide-based compound. Examples of the solvent for the preparation include water, ethanol, and glycerin. Examples of the excipient include lactose, sucrose, glucose, mannitol, sorbitol, corn starch, potato starch, α-starch, dextrin, carboxymethyl starch, crystalline cellulose, hydroxypropyl methyl cellulose, carboxymethyl cellulose, carboxymethyl cellulose calcium, gum arabic, dextran, pullulan, silicates, calcium phosphate, calcium carbonate, calcium sulfate and the like. Examples of the silicates include light anhydrous silicic acid, synthetic aluminum silicate, magnesium aluminometasilicate and the like. Examples of the binding agent include gelatin, a polyvinyl pyrrolidone, macrogol and the like. Examples of the disintegrant include croscarmellose sodium, sodium carboxymethyl starch, a cross-linked polyvinyl pyrrolidone and the like. Examples of the lubricating agent include talc, stearic acid, calcium stearate, magnesium stearate, colloidal silica, Veegum, beeswax, spermaceti, boric acid, glycol, fumaric acid, adipic acid, sodium benzoate, sodium sulfate, leucine, sodium lauryl sulfate, magnesium lauryl sulfate, silicic anhydride, silicic acid hydrate and the like. Examples of the stabilizer include methylparaben, propylparaben, chlorobutanol, benzyl alcohol, phenylethyl alcohol, benzalkonium chloride, phenol, cresol, thimerosal, acetic anhydride, sorbic acid and the like. Examples of the suspending agent include polysorbate 80, sodium carboxymethyl cellulose and the like.

### 4. Resin composition and resin molded product having antibacterial properties

The antibacterial composition of the present invention can also be used for a resin composition, a resin molded product molded from the composition, a coating material, and the like. In addition, the resin composition containing the antibacterial composition of the present invention and the resin molded product molded from the composition can be surface-treated with a known modifier, for example, a dispersant, a surfactant, a coupling agent, an anti-discoloration agent, an antioxidant, an ultraviolet absorber, and the like as long as the performance is not impaired. As the dispersant, for example, waxes or low melting point resins are used. As the surfactant, a known cationic or anionic surfactant, a known nonionic surfactant, and a known amphoteric surfactant can be generally used. As the coupling agent, for example, a known silane-based coupling agent or titanate-based coupling agent can be generally used. The antibacterial composition of the present invention can be used in combination with other organic antibacterial agents, bactericides, and preservatives.

The resin is not particularly limited, and a wide range of resins such as a polyolefin resin, a polyvinyl chloride resin, polyvinylidene chloride, a chlorine-based resin such as a chlorinated polyolefin, polyamide, an ABS resin and the like can be used.

Examples of the material of the coating material with which the antibacterial composition of the present invention is blended include oily coating materials such as boil oil, oil varnish, and oily enamel, fiberderivative coating materials such as nitrocellulose lacquer and acrylic lacquer, and synthetic resin coating materials in which the thermosetting resin, elastomer polymer, and the like described above is used as a coating material.

Materials conventionally used can also be used as materials used by being blended with the resin or coating material of the present invention. Examples thereof include fats and oils, mineral oils, a plasticizer, a solvent, an inorganic filler, a pigment, an extender pigment and the like. Examples of the inorganic filler include fine powder silica, active alumina, aluminumcontaining zinc phyllosilicate and a silica-like composite thereof, magnesium phyllosilicate, hydrotalcite, zinc-modified hydrotalcite, lithiumaluminum composite hydroxide salt, talc, clay, bentonite, dosonite, diatomaceous earth, silica sand, calcium carbonate and the like.

Examples of the resin molded product or coating material blended with the antibacterial composition of the present invention include those having any shape. Examples thereof include cloth products such as woven fabric, nonwoven fabric, net fabric, knitted fabric and the like, sheet products such as paper, a film and the like, powder products such as a spray, a spraying agent and the like, liquid or paste products such as a brush coating material, a spray coating material, a roller coating material, an adhesive, a sealant and the like, and tool-shaped molded products such as a plate, a rod, a box, a porous body and the like.

### Examples

Hereinafter, the present invention will be specifically described based on Examples, but these Examples do not limit the content of the present invention. In addition, in the following Examples, the expression "%" is based on mass (mass percentage) unless otherwise specified.

### 1. Methods

### (1) Bacterial strains and reagents

Each strain of E. faecium (JNBP_01969), C. perfringens (str. 13), and C. difficile (ATCC9689) was tested. An E. faecium strain was cultured in NATY medium (1.5% NaCl, 2% tryptone (NACALAI TESQUE, INC.), 1% yeast extract (NACALAI TESQUE, INC.), 0.85% Na₂HPO₄, and 1% glucose). A C. difficile strain and a C. perfringens strain were cultured in BHIS medium (2% NaCl, 1.85% BACTO Brain heart infusion (BD), 0.25% Yeast extract (NACALAI TESQUE, INC.), and 0.03% L-cysteine (NACALAI TESQUE, INC.)). A pH of each of NaTY medium and BHIS medium was adjusted with MES (pH 6.0 and pH 6.5), HEPES (pH 7.0, 7.5, and 8.0), or tricine (pH 8.5). Vancomycin, ampicillin, cefoperazone, linezolid, niclosamide, and oxyclozanide were purchased from Pfizer Inc., NACALAI TESQUE, INC., Teva Takeda Yakuhin Ltd., FUJIFILM Wako Pure Chemical Corporation, Sigma-Aldrich, and AdooQ BioScience, respectively.

### (2) Inhibition of proliferation of E. faecium

An E. faecium strain was pre-cultured in NaTY medium to which 4 µg/ml vancomycin was added at 37°C for 24 hours. 4 µg/mL vancomycin was additionally added to a medium for VRE. A primary suspension was diluted with NaTY medium to which 4 µg/ml of vancomycin was added and adjusted to a bacterial concentration of about 1 × 10⁶ CFU/ml. A compound was dispensed into culture tubes and 5 mL of NaTY medium (various pH) containing 4 ug/ml vancomycin and 50 µL of a secondary suspension were added to each tube. Thereafter, the tube was cultured at 37°C for 24 hours and monitored every 30 minutes using OD-monitor C&T (TAITEC CORPORATION).

### (4) Effect of inhibiting proliferation of C. perfringens and C. difficile

A C. perfringens strain and a C. difficile strain were cultured anaerobically in BHIS medium at 37°C for 24 hours. A primary suspension was diluted with BHIS medium and a bacterial concentration was adjusted to about 1 × 10⁶ CFU/ml. A compound was dispensed into culture tubes, and 3 mL of BHIS medium (various pH) and 30 µL of a secondary suspension were added to each tube. Thereafter, the tube was cultured anaerobically at 37°C for 24 hours and monitored every 1 hour using OD-monitor C&T (TAITEC CORPORATION).

### (5) NMR spectrum

¹H NMR spectra were measured at ambient temperature with JEOL ECS-500 (500 MHz) spectrometer. Data were recorded as follows: chemical shifts (ppm) from internal tetramethylsilane on the δ scale, multiplicities (s = singlet; d = doublet; t = triplet; q = quartet; sep = septet; m = multiplet; br = broad), binding constants (Hz), integration, and assignments.

¹³C NMR spectra were measured with ECS-500 (125 MHz) spectrometer of JEOL Ltd. Chemical shifts were recorded in ppm from solvent resonance (deuterated water chloroform: 77.0 ppm) used as internal standard. Infrared (IR) spectra were recorded with JASCO FT/IR 4100 spectrometer. A precoated TLC plate (silica gel 60GF254 0.25 mm) from Merck was used for thin layer chromatography (TLC) analysis. Visualization was performed by ultraviolet rays (254 nm), anisaldehyde, KMnO₄, and phosphomolybdic acid. In experiments where dry DMF was required, DMF was distilled prior to use.

### (6) Procedure for synthesis of N,N-dimethyl-4-(5-methyl-1H-benzo[d]imidazol-2-yl)aniline (V-161)

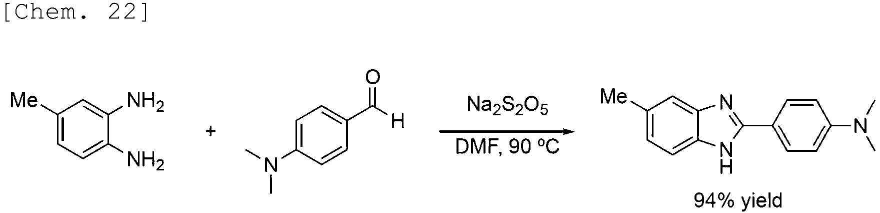

To a solution of 3,4-diaminotoluene (2.57 g, 21.0 mmol) and 4-dimethylaminobenzaldehyde (2.98 g, 20.0 mmol) in DMF (40 mL), Na₂S₂O₅ (3.80 g, 20.0 mmol) was added at room temperature. The reaction mixture was stirred at 90°C for 12 hours. The reaction mixture was cooled to room temperature and poured into ice-water (266 mL). The resulting suspension was stirred for 4 hours and then cooled to 0°C. A precipitated solid was washed with cold water (50 mL) and dried under reduced pressure to obtain V-161 (4.99 g, 94% yield) as a target product.

N,N-dimethyl-4-(5-methyl-1H-benzo[d]imidazol-2-yl)aniline: white solid, ¹H NMR (500 MHz, methanol-d₄) 57.82 (d, J = 9.0 Hz, 2H), 7.37 (d, J = 8.0 Hz, 1H), 7.28 (s, 1H), 6.99 (d, J = 8.0 Hz, 1H), 6.73 (d, J = 9.0 Hz, 2H), 2.93 (s, 7H), 2.40 (s, 3H).

¹³C NMR (125 MHz, methanol-d₄) δ153.9, 153.2, 139.5, 137.9, 133.4, 128.8 (2C), 124.9, 117.4, 114.9, 114.7, 113.0 (2C), 40.3 (2C), 21.7.

IR(neat)2917, 1609, 1442, 1364, 1205, 1065 cm⁻¹.

### (7) Method for producing N,N-dimethyl-4-(5-methyl-1H-benzo[d]imidazol-2-yl)aniline hydrochloride

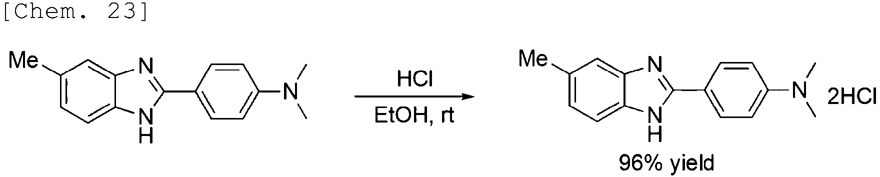

N,N-dimethyl-4-(5-methyl-1H-benzo[d]imidazol-2-yl)aniline (4.91 g, 19.5 mmol) was dissolved in ethanol (115 mL), and the mixture was reacted at room temperature in a chlorine gas atmosphere. After 2 hours, the reaction solution was removed, and the obtained solid was filtered with diethyl ether to obtain N,N-dimethyl-4-(5-methyl-1H-benzo[d]imidazol-2-yl)aniline hydrochloride (6.06 g, 96% yield).

### (8) Mice

Six-week-old ICR female mice were purchased from Japan SLC, Inc. The purchased mice were transferred to individual isolators in the animal house at the Medical Mycology Research Center, Chiba University. These mice were fed sterilized feed (CL-2, LEA Japan, Inc.) and water, and were kept in a specific pathogen free (SPF) environment using a cage with an autoclaved bed. The present experiment using mice has been approved by the Institutional Animal Care and Use Committee of Chiba University.

### (9) Vancomycin-resistant Enterococcus Faecium (VRE) infection model

0.5 g/L of cefoperazone (MP Biomedicals) was added to and dissolved in the drinking water for the purchased ICR mice, and the mice were administered with free drinking for 1 week. Feed and water were removed 3 hours before oral administration of VRE, and the mice were fed feed and water containing cefoperazone after VRE and drug administration.

VRE (JNBP_01969) purchased from American Type Culture Collection (ATCC) was seeded on a VRE selection medium plate (BD), and cultured in an incubator at 37°C for 24 hours. The observed colonies were cultured overnight at 37°C using 2.5 mL of 2X NaTY medium (2% tryptone, 1% yeast extract, 1.7% Na₂HPO₄, and 1% glucose) to which 5 µg/mL vancomycin (Shionogi & Co., Ltd.) was added. The cultured VRE was subcultured in 2.5 mL of new NaTY medium to which 5 µg/mL vancomycin (Shionogi & Co., Ltd.) was added on the day of oral administration, and was cultured at 37°C for about 2.5 hours. VRE was collected at OD₆₀₀ = 1 hour and 1 × 10⁶ colony-forming units (CFU) per mouse were orally administered using afeeding needle.

### (10) V-161 and niclosamide oral administration

1 hour after oral administration of VRE, capsules (Porcine Hard Gelatin Capsules Size M, Torpac Inc.) containing 0.9 mg of V-161 hydrochloride and 0.3 mg of niclosamide, or V-161 hydrochloride, or niclosamide were orally administered to each mouse. These drugs were administered orally twice a day for 3 days, and the feces of the mice were collected 4 hours after the last oral administration. Furthermore, the mice were euthanized by cervical dislocation, and then dissected, and the contents of the small intestine and the large intestine were collected.

### (11) Verification of inhibitory effect of V-161 and niclosamide on VRE intestinal colonization

The weight of each of the collected small intestine, large intestine contents, and feces was measured, and sterilized water was added so as to have a concentration of 0.1 g/mL for homogenization. Each sample was serially diluted, and 100 mL of the sample was applied to a VRE selection media plate (BD) and cultured at 37°C for 48 hours to count the number of grown colonies.

### 2. In vitro proliferation inhibitory effect of E. faecium using V-161 and niclosamide (NIC) in combination

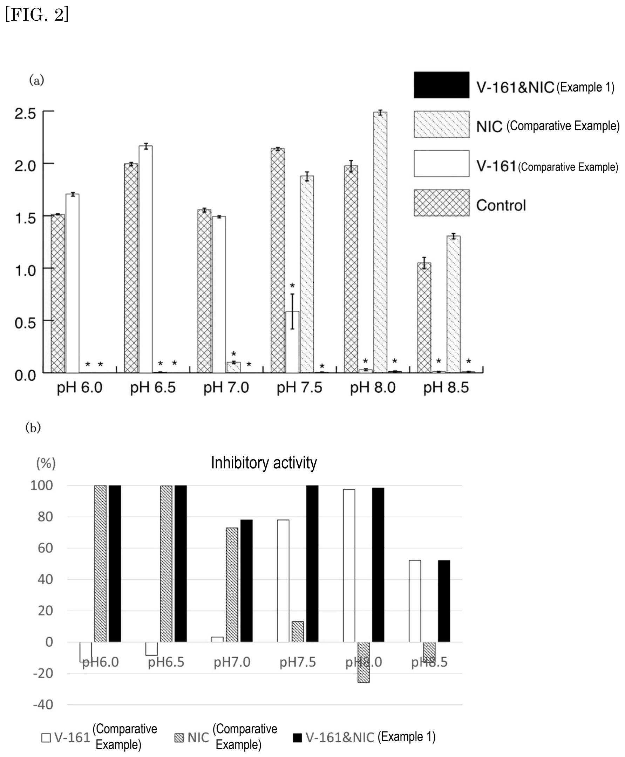

Fig. 2 is a graph showing the results of the in vitro proliferation inhibitory effect of E. faecium using V-161 in combination with niclosamide. 10 µg/mL V-161 and 1 µg/mL niclosamide in vancomycin resistant E.faecium (VRE) were added to NaTY medium and cultured at the indicated pH at 37°C (13 hours). Bacterial proliferation was determined by measuring OD₆₀₀. All data represent the mean ± standard error of the mean (SEM) of three independent experiments. Symbols indicate statistical significance (Student's t-test, * = p < 0.01). In Fig. 2(a), the vertical axis represents the OD₆₀₀ value, and a lower value indicates a higher proliferation inhibitory effect. In Fig. 2(b), the value (percentage) obtained by dividing the value obtained by subtracting the value of each example from the value of Control by the value of Control is plotted on the vertical axis, and a higher the numerical value indicates a higher proliferation inhibitory effect. As can be seen from the drawing, when V-161 and niclosamide were used in combination (V-161&NIC: Example 1 in the drawing), proliferation of VRE was inhibited in the entire range of a pH of 6.0 to 8.5. Particularly, at a pH of 7.5, the proliferation of bacteria was not completely inhibited when each of V-161 and niclosamide was used alone (each in Comparative Example), whereas the proliferation of bacteria was completely inhibited when both were used in combination (Example 1). This is presumed to be because the proliferation of bacteria was inhibited by the synergistic effect of both. Such a synergistic effect is also observed at a pH of 7.0.

### 3. In vitro proliferation inhibitory effect of E. faecium in coexistence of antibiotics

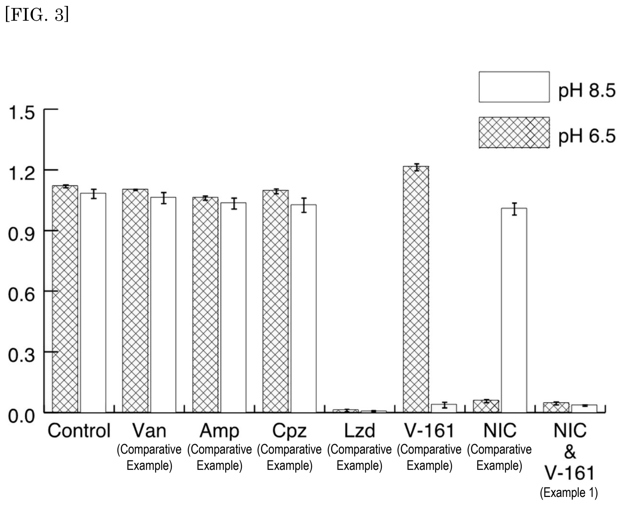

Fig. 3 is a graph showing the results of the in vitro proliferation inhibitory effect of E. faecium in the coexistence of antibiotics. Dimethyl sulfoxide (DMSO) as a VRE culture control, 10 µg/mL vancomycin (Van), 10 µg/mL ampicillin (Amp), 10 µg/mL cefoperazone (Cpz), 10 µg/mL linzolide (Lzd), 10 µg/mL niclosamide (NIC), 10 µg/mL V-161, and 10 µg/mL niclosamide (each in Comparative Example), and a combination of 10 µg/mL V-161 (NIC&V-161 in the drawing: Example 1) were incubated in NaTY medium at the indicated pH at 37°C (24 hours). Bacterial proliferation was determined by measuring OD₆₀₀. All data represent the mean ± standard error of the mean (SEM) of three independent experiments. As can be seen from the drawing, when V-161 and niclosamide were used in combination (Example 1), proliferation of VRE was inhibited at both of pH 6.5 and pH 8.5.

### 4. Synthesis of V-161 analogue

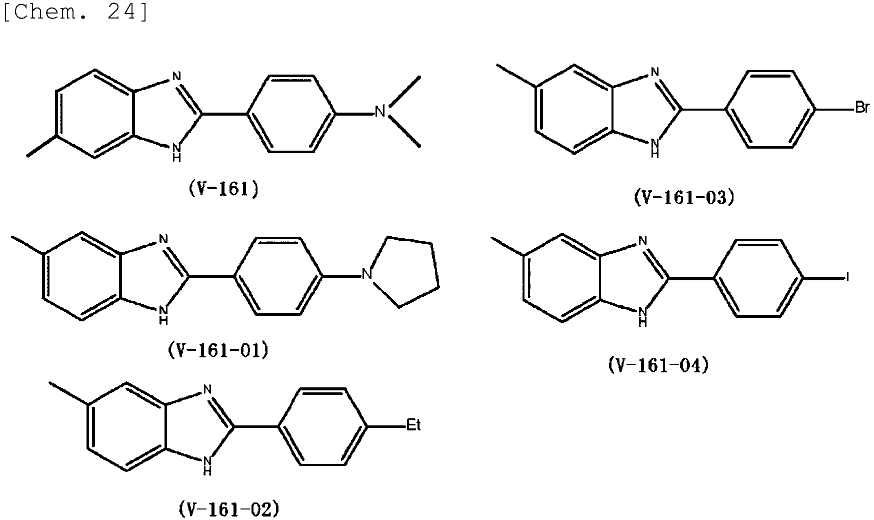

### (1) Procedure for synthesis of 5-methyl-2-(4-(pyrrolidin-1-yl)phenyl-1H-benzo[d]imidazole (V-161-01)

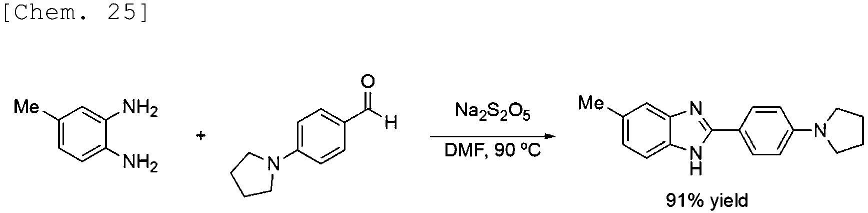

To a solution of 3,4-diaminotoluene (64.1 mg, 0.525 mmol) and 4-(1-pyrrolidino)benzaldehyde (87.6 mg, 0.50 mmol) in DMF (1 mL), Na₂S₂O₅ (95.1 mg, 0.50 mmol) was added at room temperature. The reaction mixture was stirred at 90°C for 12 hours. The reaction mixture was cooled to room temperature and poured into ice-water (6.7 mL). The resulting suspension was stirred for 4 hours and then cooled to 0°C. A precipitated solid was washed with cold water (10 mL) and dried under reduced pressure to obtain V-161-01 (102.5 mg, 91% yield) as a target product.

5-Methyl-2-(4-(pyrrolidin-1-yl)phenyl-1H-benzo[d]imidazole: white solid, ¹H NMR(500 MHz, DMSO-d₆)δ 7.96(d, J = 9.0 Hz, 2H),7.44(d, J = 8.0 Hz, 1H), 7.35(s, 1H), 7.06(d, J = 8.0 Hz, 1H), 6.69(d, J = 9.0 Hz, 2H), 3.33(d, J = 6.5 Hz, 4H), 2.43(s, 3H), 2.02-1.95(m, 4H).

¹³C NMR(125 MHz,DMSO-d₆)δ151.0, 149.2, 136.4, 134.8, 131.7, 127.9(2C), 123.7, 113.4, 113.1, 111.5(3C), 47.1(2C), 24.6(2C), 20.9.

IR(neat)2858, 1606, 1457, 1392, 1210, 1060 cm⁻¹.

### (2) Procedure for synthesis of 2-(4-ethylphenyl)-5-methyl-1H-benzo[d]imidazole (V-161-02)

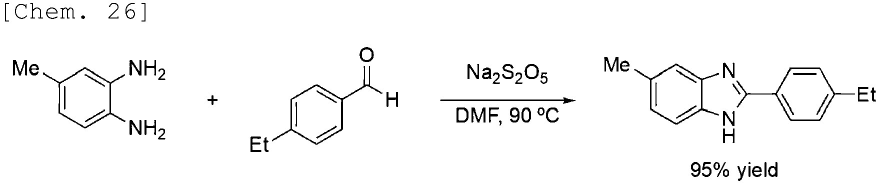

To a solution of 3,4-diaminotoluene (128.3 mg, 1.05 mmol) and 4-ethylbenzaldehyde (134.2 mg, 1.0 mmol) in DMF (2 mL), Na₂S₂O₅ (190.11 mg, 1.0 mmol) was added at room temperature. The reaction mixture was stirred at 90°C for 12 hours. The reaction mixture was cooled to room temperature and poured into ice-water (6.7 mL). The resulting suspension was stirred for 4 hours and then cooled to 0°C. A precipitated solid was washed with cold water (10 mL) and dried under reduced pressure to obtain V-161-02 (225.5 mg, 95% yield) as a target product.

2-(4-Ethylphenyl)-5-methyl-1H-benzo[d]imidazole: white solid, ¹H NMR(500 MHz, DMSO-d₆)δ8.06(d, J = 8.2 Hz, 2H), 7.48(d, J = 8.0 Hz, 1H), 7.38(d, J = 8.0 Hz, 2H), 7.37(s, 1H), 7.05(d, J = 8.0 Hz, 1H), 2.69(q, J = 7.5 Hz, 2H), 2.43(s, 3H), 1.23(t, J = 7.5 Hz, 3H).

¹³C NMR(125 MHz, DMSO-d₆)δ150.6, 145.8, 138.1, 136.7, 131.4, 128.0(2C), 126.7, 126.3(2C), 123.6, 114.4, 113.9, 27.7, 21.0, 14.8.

IR(neat)2965, 1632, 1445, 1392, 1230, 1067 cm⁻¹.

### (3) Procedure for synthesis of 2-(4-bromophenyl)-5-methyl-1H-benzo[d]imidazole (V-161-03)

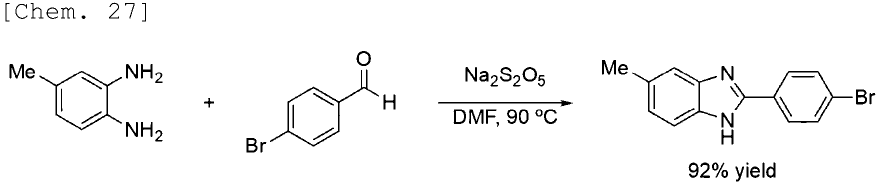

To a solution of 3,4-diaminotoluene (64.1 mg, 0.525 mmol) and 4-bromobenzaldehyde (92.5 mg, 0.50 mmol) in DMF (1 mL), Na₂S₂O₅ (95.1 mg, 0.50 mmol) was added at room temperature. The reaction mixture was stirred at 90°C for 12 hours. The reaction mixture was cooled to room temperature and poured into ice-water (6.7 mL). The resulting suspension was stirred for 4 hours and then cooled to 0°C. A precipitated solid was washed with cold water (10 mL) and dried under reduced pressure to obtain V-161-03 (132.0 mg, 92% yield) as a target product.

2-(4-Bromophenyl)-5-methyl-1H-benzo[d]imidazole: white solid, ¹H NMR(500 MHz, DMSO-d₆)δ8.08(d, J = 8.5 Hz, 2H), 7.77(d, J = 8.5 Hz, 2H), 7.52(d, J = 8.2 Hz, 1H), 7.42(s, 1H), 7.11(d, J = 8.2 Hz, 1H), 2.44(s, 3H).

¹³C NMR(125 MHz, DMSO-d₆)δ149.2, 137.4, 136.0, 132.4, 131.8(2C), 128.3(2C), 127.8, 124.4, 123.6, 114.6, 113.9, 21.0.

IR(neat)2860, 1631, 1470, 1379, 1232, 1070 cm⁻¹.

### (4) Procedure for synthesis of 2-(4-iodophenyl)-5-methyl-1H-benzo[d]imidazole (V-161-04)

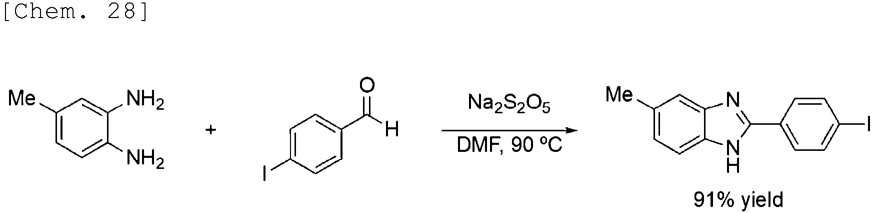

To a solution of 3,4-diaminotoluene (64.1 mg, 0.525 mmol) and 4-iodobenzaldehyde (116.0 mg, 0.50 mmol) in DMF (1 mL), Na₂S₂O₅ (95.1 mg, 0.50 mmol) was added at room temperature. The reaction mixture was stirred at 90°C for 12 hours. The reaction mixture was cooled to room temperature and poured into ice-water (6.7 mL). The resulting suspension was stirred for 4 hours and then cooled to 0°C. A precipitated solid was washed with cold water (10 mL) and dried under reduced pressure to obtain V-161-04 (151.3 mg, 91% yield) as a target product.

2-(4-Iodophenyl)-5-methyl-1H-benzo[d]imidazole: white solid, ¹H NMR(500 MHz, DMSO-d₆)δ7.99-7.94(m, 2H), 7.94-7.90(m, 2H), 7.53(d, J = 8.2 Hz, 1H), 7.43(s, 1H), 7.13(d, J = 8.2 Hz, 1H), 2.45(s, 3H).

¹³C NMR(125 MHz, DMSO-d₆)δ 149.3, 137.7(2C), 137.1, 135.6, 132.6, 128.3(2C), 127.7, 124.5, 114.5, 113.9, 97.1, 21.0.

IR(neat)2861, 1631, 1470, 1379, 1232, 1060 cm⁻¹.

V-161-01, V-161-02, V-161-03, and V-161-04 were synthesized by the above method.

### (5) In vitro proliferation inhibitory effect of E. faecium using V-161 or analogue thereof in combination with niclosamide

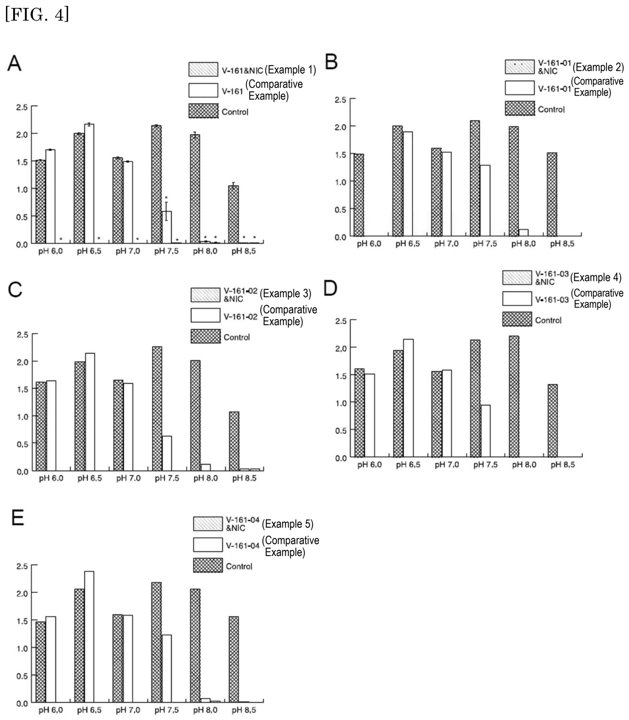

Fig. 4 is a graph showing the in vitro proliferation inhibitory effect of E. faecium using V-161 or an analogue thereof in combination with niclosamide. 40 µM (10 µg/mL) V-161 (A), 20 µM V-161-01 (B), 20 µM V-161-02 (C), 20 µM V-161-03 (D), 20 µM V-161-04 (E), and 1 µg/mL niclosamide were added in combination to NaTY medium, and E. faecium was cultured at the indicated pH at 37°C (12 to 18 hours). "V-161-01&NIC" to "V-161-04&NIC" in the drawing are "Example 2" to "Example 5", respectively. Bacterial proliferation was determined by measuring OD₆₀₀. As can be seen from the drawing, when any analogue was used in combination with niclosamide, the proliferation of VRE was inhibited over the entire range of a pH of 6.0 to 8.5.

### 3. In vitro proliferation inhibitory effect of E. faecium using salicylanilide-based compound in combination with V-161

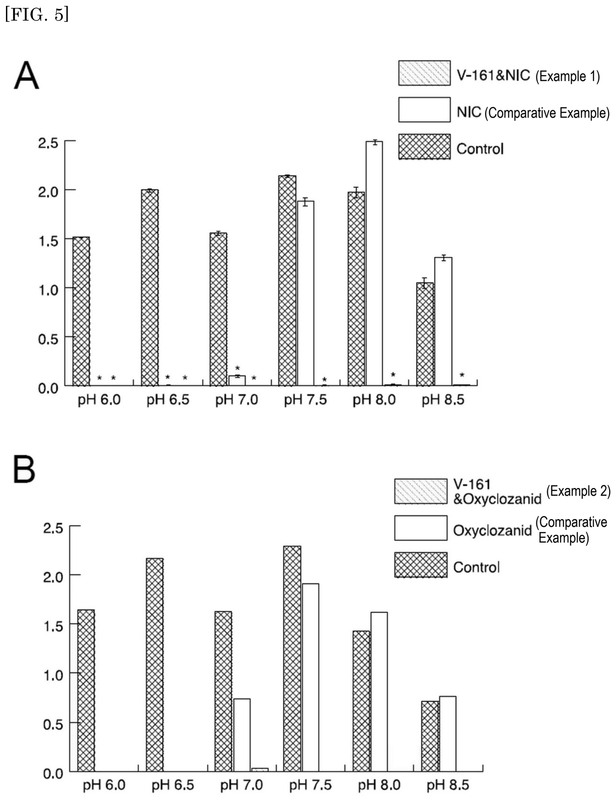

Fig. 5 is a graph showing the results of the in vitro proliferation inhibitory effect of E. faecium using a salicylanilide-based compound in combination with V-161. 1 µg/mL niclosamide (A), 1 µg/mL oxyclozanide (B), and 10 µg/mL V-161 were added in combination to NaTY medium, and E. faecium was cultured at the indicated pH at 37°C (10 to 12 hours). Bacterial proliferation was determined by measuring OD₆₀₀. As can be seen from the drawing, also when V-161 and oxyclozanide were used in combination (Example 6), the proliferation of VRE was inhibited over the entire range of a pH of 6.0 to 8.5, similarly to the case where V-161 and niclosamide were used in combination (Example 1).

### 4. In vitro proliferation inhibitory effect by combination use of V-161 of C. perfringens and C. difficile and niclosamide

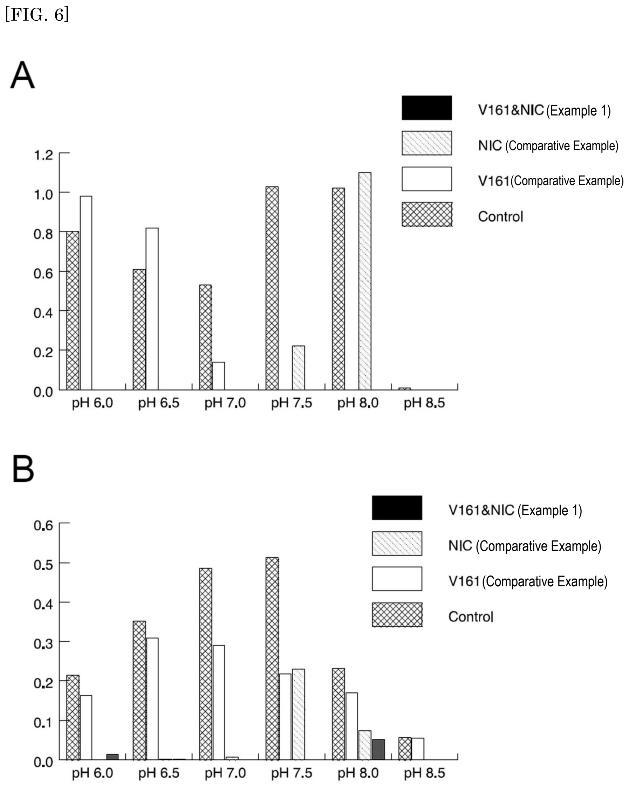

Fig. 6 is a graph showing the results of the in vitro proliferation inhibitory effect of combination use of V-161 of C. perfringens and C. difficile and niclosamide. C. perfringens (A) and C. difficile (B) were added in combination with 32 µg/mL niclosamide and 25 µg/mL V-161 to BHIS medium and cultured at 37°C under anaerobic conditions at the indicated pH (5 to 6 hours). Bacterial proliferation was determined by measuring OD₆₆₀. As can be seen from the drawing, when V-161 and niclosamide were used in combination (Example 1), proliferation of C. perfringens and C. difficile was inhibited over the entire range of a pH of 6.0 to 8.5.

### 5. Verification of inhibitory effect of V-161 and niclosamide on VRE intestinal colonization

V-161, niclosamide, and V-161 and niclosamide, were orally administered to mice pre-administered with VRE. The number of VREs (colony forming unit (CFU)) in the small intestine contents after 12 hours was measured.

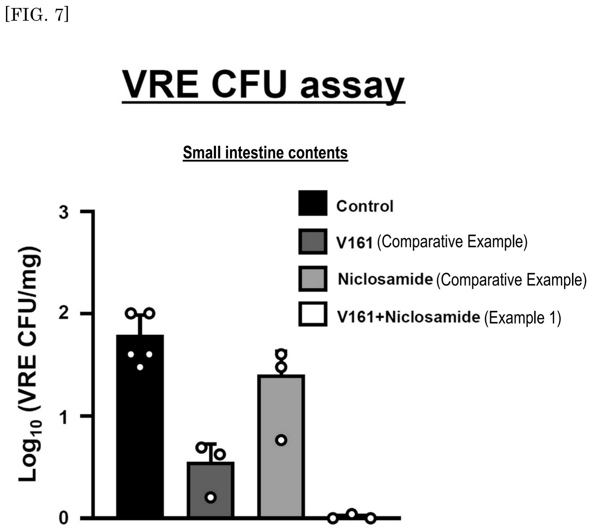

Fig. 7 is a graph showing the in vivo proliferation inhibitory effect of VRE by V-161, niclosamide, and V-161 and niclosamide, orally administered to the mice to which VRE is previously administered. As can be seen from the drawing, when V-161 and niclosamide were used in combination (Example 1), the number of VREs was significantly reduced as compared with the case where each was used alone (Comparative Example), and the proliferation of VRE in the small intestine was remarkably inhibited.

## Claims

1. An antibacterial composition comprising a 2-phenylbenzimidazole-based compound and a salicylanilide-based compound.

2. The antibacterial composition according to claim 1, wherein the 2-phenylbenzimidazole-based compound is a compound represented by the following Formula (1): 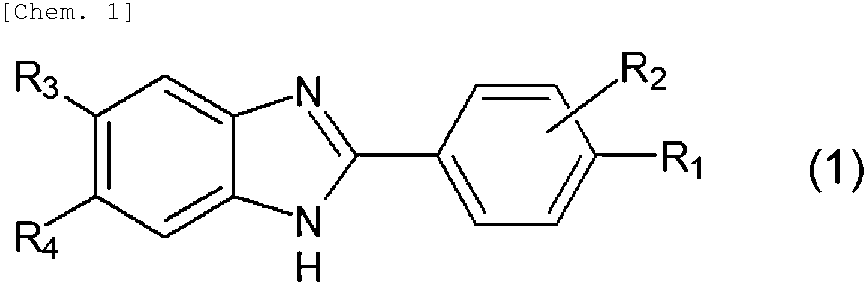 where R₁ is selected from a hydroxy group, an alkyl group having 1 to 10 carbon atoms, an alkoxy group having 1 to 10 carbon atoms, and a haloalkoxy group having 1 to 3 carbon atoms, each bonded to an adjacent phenyl group via oxygen, or selected from a dialkylamino group having 1 to 10 carbon atoms of each alkyl group, a heterocyclic amine having 2 to 6 carbon atoms, and a carboxylic acid amide group which may have a substituent bonded to a carbon atom, each bonded to an adjacent phenyl group via nitrogen, or represents bromine, iodine, or a straight-chain hydrocarbon group having 2 to 5 carbon atoms, R₂ represents hydrogen or a haloalkoxy group having 1 to 3 carbon atoms, R₃ and R₄ represent a hydrogen atom, an alkyl group having 1 to 4 carbon atoms, or a halogen atom selected from fluorine, chlorine, bromine, and iodine, and R₃ and R₄ may be the same as or different from each other.

3. The antibacterial composition according to claim 2, wherein the compound represented by Formula (1) is selected from the group consisting of compounds represented by the following Formulas (1-1) to (1-5).

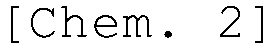

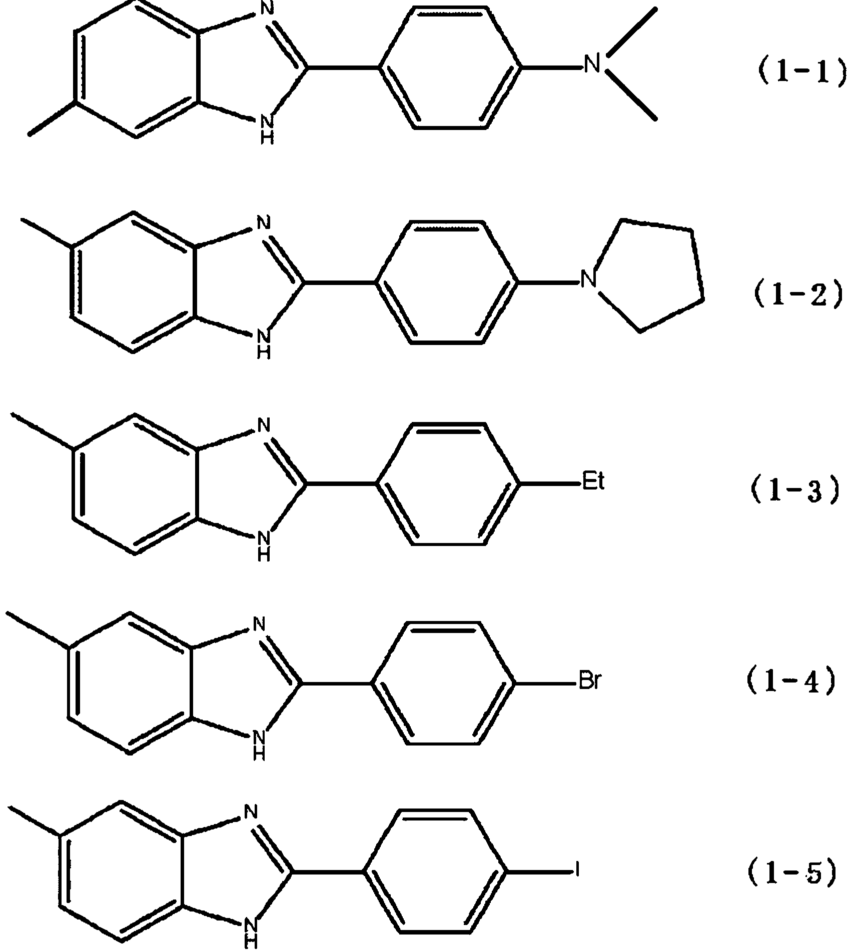

4. The antibacterial composition according to claim 1, wherein the salicylanilide-based compound is a compound represented by the following Formula (2): 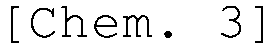 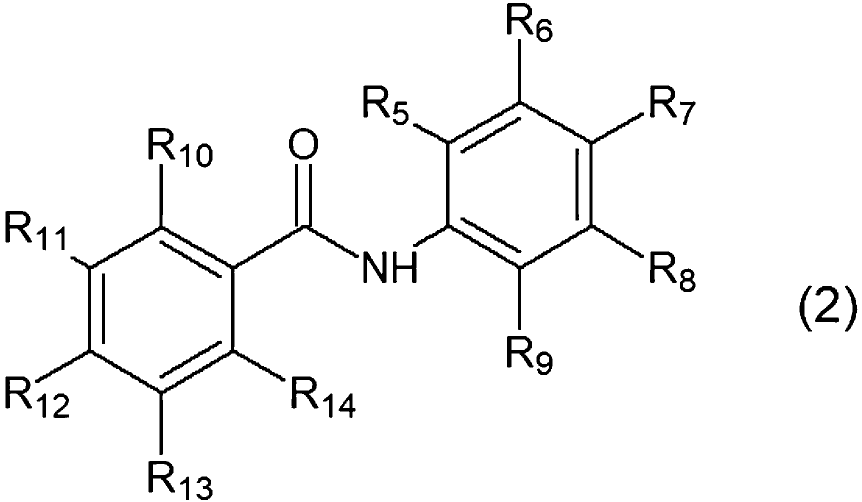 where R₃ to R₉ each represent a hydrogen atom, a nitro group, or a halogen atom selected from fluorine, chlorine, bromine, and iodine, at least one of R₅ to R₉ is a halogen atom, R₃ to R₉ may be the same as or different from each other, R₁₀ to R₁₄ represent a hydrogen atom, a hydroxyl group, or a halogen atom selected from fluorine, chlorine, bromine, and iodine, at least one of R₁₀ to R₁₄ is a hydroxyl group, at least one is a halogen atom, and R₁₀ to R₁₄ may be the same as or different from each other.

5. The antibacterial composition according to claim 4, wherein the compound represented by Formula (2) is niclosamide represented by the following Formula (2-1) or oxyclozanide represented by the following Formula (2-2).

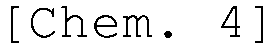

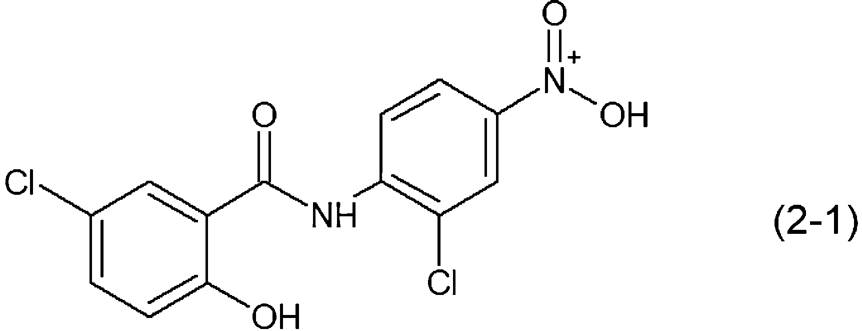

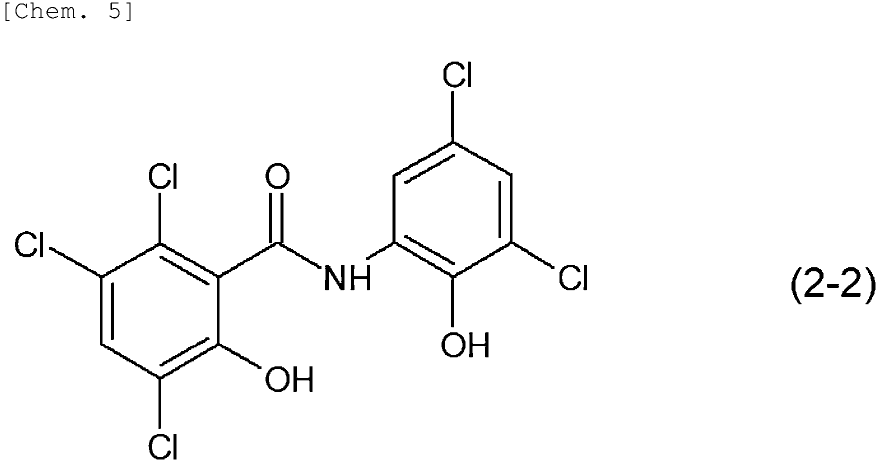

6. A pharmaceutical reagent comprising the antibacterial composition according to claim 1.

7. An antibacterial method for inhibiting proliferation of bacteria using the antibacterial composition according to claim 1, the method comprising:
administering the 2-phenylbenzimidazole-based compound and the salicylanilide-based compound to bacteria having Na⁺-transporting V-ATPase and F-ATPase; and
binding the 2-phenylbenzimidazole-based compound to the V-ATPase of the bacteria to inhibit activity of the V-ATPase, and breaking a proton concentration gradient by the F-ATPase with the salicylanilide-based compound to inhibit activity of the F-ATPase.

8. The antibacterial method according to claim 7, wherein the bacteria are drug-resistant enterococci.

9. Use of a 2-phenylbenzimidazole-based compound for combined use with a salicylanilide-based compound.

10. The use according to claim 9, wherein the 2-phenylbenzimidazole-based compound is a compound represented by the following Formula (1).

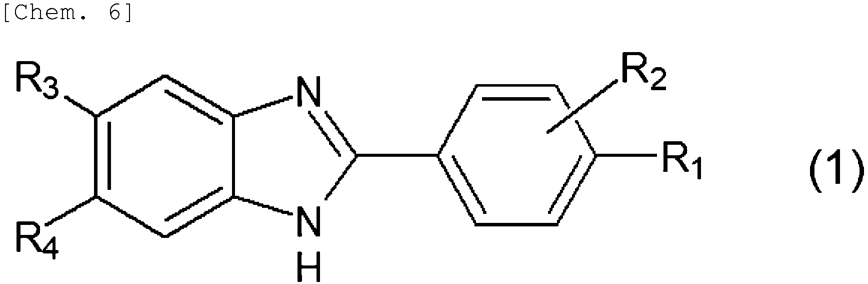

11. The use according to claim 10, wherein the compound represented by Formula (1) is selected from the group consisting of compounds represented by the following Formulas (1-1) to (1-5).

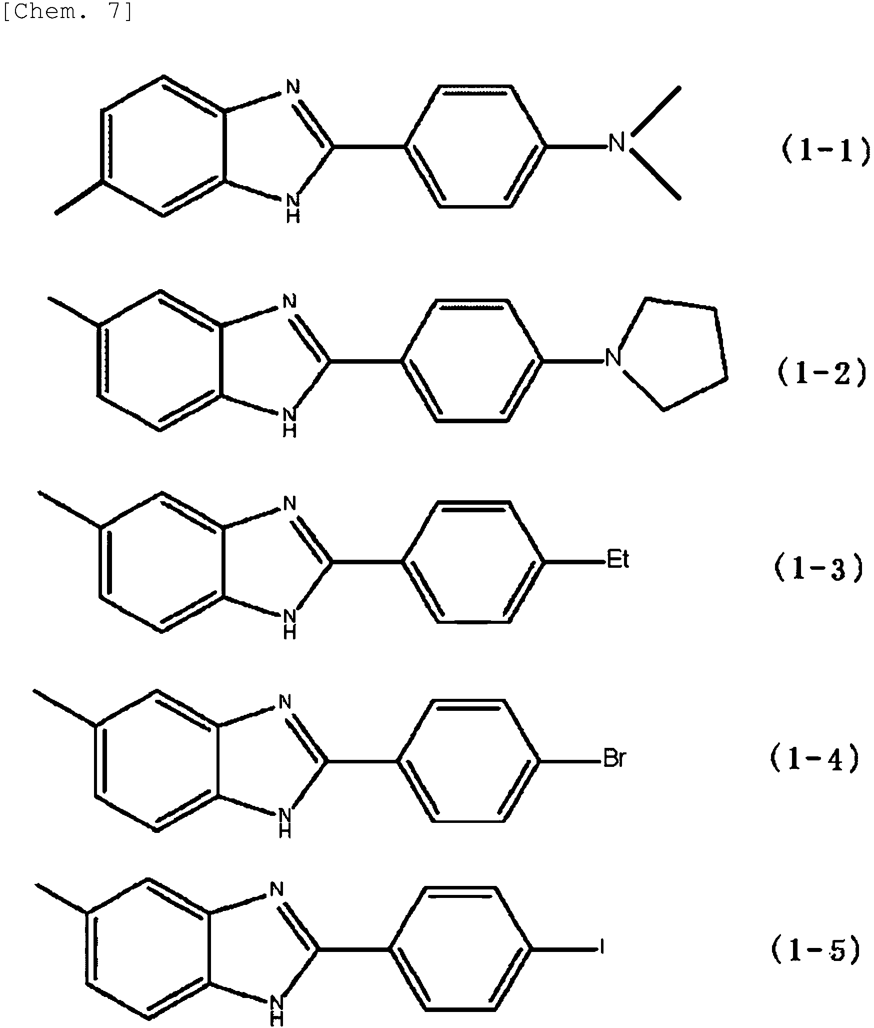

12. Use of a salicylanilide-based compound for combined use with a 2-phenylbenzimidazole-based compound.

13. The use according to claim 12, wherein the salicylanilide-based compound is a compound represented by the following Formula (2).

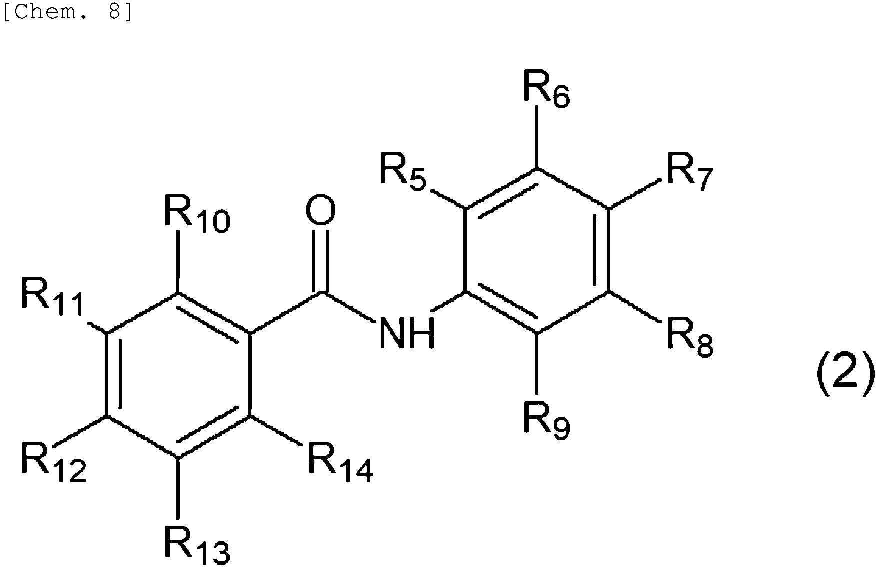

14. The use according to claim 13, wherein the compound represented by Formula (2) is niclosamide represented by the following Formula (2-1) or oxyclozanide represented by the following Formula (2-2).
